# EUROPEAN PATENT APPLICATION

(11) **EP 4 840 420 A1**
(43) Date of publication of application: **18.11.2026**
(21) Application number: 26179022.4
(22) Date of filing: 15.05.2026
(51) Int. Cl.: A61C 7/00, A61C 7/08

(54) **LANDMARKS FOR TRACKING CHANGES IN ORTHODONTIC ALIGNERS**

(30) Priority: 16.05.2025 US 202519210737
(71) Applicant: Patel, Bharat, York, Pennsylvania 17401 (US); Terry, Andrea Q., Mooresville, North Carolina 28117 (US); Hurlburt, Catherine M., Charlotte, North Carolina 28208 (US); Tripp, Isaac James, Boston, Massachusetts 02111 (US); Fehr, Jean-Noel, 2000 Neuchâtel (CH); Ulrich, Dieter Albert, 8049 Zurich (CH)
(72) Inventor: Patel, Bharat, York, Pennsylvania 17401 (US); Terry, Andrea Q., Mooresville, North Carolina 28117 (US); Hurlburt, Catherine M., Charlotte, North Carolina 28208 (US); Tripp, Isaac James, Boston, Massachusetts 02111 (US); Fehr, Jean-Noel, 2000 Neuchâtel (CH); Ulrich, Dieter Albert, 8049 Zurich (CH)
(74) Representative: Aldridge, Henry Alexander

(57) **Abstract**

An orthodontic aligner tray comprises a shell having a plurality of tooth receiving cavities shaped to apply a repositioning force to teeth of a patient, and a plurality of landmarks present on the shell, wherein the plurality of landmarks are configured to be detected to determine a shape of the shell. A method of tracking movement of teeth from application of an orthodontic aligner tray comprises designing the aligner tray to have landmarks disposed at a first position within a three-dimensional coordinate system for the aligner tray, receiving electronic files of the aligner tray after being applied to teeth so that the aligner tray is deformed to move at least some of the landmarks into a second position, determining changes in locations of the landmarks in the three-dimensional coordinate system from the first position to the second position from the electronic files, and outputting an indication of the changes.

## Description

### TECHNICAL FIELD

The present disclosure is generally directed to, but not by way of limitation, systems, devices and methods for performing orthodontic procedures, such as those related to using orthodontic aligner trays. More specifically, but not by way of limitation, the present disclosure is directed to systems, devices and methods for tracking and monitoring progress of orthodontic procedures.

### BACKGROUND

The field of orthodontics relates to the treatment of misalignment and malocclusion of teeth. Conventional methods for correcting alignment of teeth involve the use of braces, brackets, wires and the like, that can be adjusted by a dental professional, such as an orthodontist, to progress through a pathway for applying appropriate forces to move the teeth into better alignment. As the teeth are moved in such conventional procedures, they will often be blocked by other teeth. Because of the initial misalignment, it is often not simply a matter of moving or repositioning a single tooth in one direction to correct its position. Rather, the treatment plan most often must account for physical contact with adjacent teeth as all of the teeth are collectively moved. For example, sometimes it is advantageous to remove small amounts of tooth matter, e.g., enamel, to facilitate alignment in a procedure called stripping or interproximal reduction (IPR). Even though effective alignment of the teeth can be ultimately achieved, some patients are not satisfied with the appearance of conventional orthodontic braces and the maintenance required of them.

More recently, methods of correcting the position of misaligned teeth involve using orthodontic aligner trays, also referred to as orthodontic aligners, orthodontic tray, aligner trays, tray aligner, aligners or tray, fabricated in a manner that a given tray will itself exert a force upon the misaligned teeth to cause movement. These include for example, the SureSmile^{®} trays from Dentsply Sirona Inc. of Charlotte, North Carolina and the Invisalign^{®} trays from Align Technology, Inc. of Santa Clara, California. Often these orthodontic aligner trays are fabricated from a clear plastic material and are disposed of after progressing to the next tray. Such orthodontic aligner trays are popular with patients for being more aesthetically pleasing and involving less maintenance.

Orthodontic aligner tray systems comprise trays that are placed over teeth to gradually reposition the teeth from a misaligned state to an aligned state. The teeth are initially scanned to generate a three-dimensional (3D) model of the misaligned teeth. The orthodontic aligner trays are derived from the 3D model using computer planning systems. The initial aligner tray can be configured to move the teeth from the position of the original 3D model. Subsequently, successive iterations of orthodontic aligner trays are planned that gradually move the teeth from the misaligned state to the aligned state based on iterative updates to the 3D model. Numerous iterations of orthodontic aligner trays can be used to achieve the final result.

As such, these orthodontic aligner trays can be provided in a series such that each successive tray moves the teeth more or differently than the previous tray, in an incremental fashion so as to affect the prescribed treatment plan. Each orthodontic aligner tray, therefore, will move certain teeth from a starting or "before" position to a selected ending or "after" position. The "after" position for a given orthodontic aligner tray can be based solely upon the nature of the immediately previous "before" position. Further, the shape and force exerted by each successive orthodontic aligner tray in the treatment process of the conventional system can be based only upon the nature of where the previous orthodontic aligner tray left off in the moving of the teeth. Thus, a treatment plan is constructed around each orthodontic aligner tray operating to perform the desired movements so that the subsequent tray is starting from the planned positions of the teeth for the subsequent tray.

Typically, there is a target goal in mind as to where the dental professional wants to ultimately move the teeth, but until the very end of the treatment procedure, this final position and the initial starting position do not affect the incremental or intermediate treatment operations. As such, it can be desirable to monitor the performance of each orthodontic aligner tray by tracking movement of teeth at each stage. However, such tracking can be burdensome. For example, tracking can sometimes involve the patient having to make an office visit to obtain appropriate measurements of the teeth by a dental professional, such as with a three-dimensional scan of the teeth, with analogue measuring instruments including gauges and calipers, or by taking impressions with subsequent scanning of the impressions. Typically, there has not been an option for a patient to obtain feedback regarding progress of the movements of the teeth in a convenient manner.

Examples of orthodontic systems are described in: Pat. No. US 9,655,691 B2 to Li et al., titled "Multilayer dental appliances and related methods and systems"; Pat. No. US 6,239,705 B1 to Glen, titled "Intra oral electronic tracking device"; Pat. No. US 10,736,715 B2 to Salah et al., titled "Dental imaging device"; Pat. No. US 11,532,079 B2 to Salah et al., titled "Dental imaging device"; Pat. No. US 11,599,997 B2 to Salah et al., titled "Dental imaging device"; Pat. No. US 11,109,945 B2 to Salah et al., titled "Method of evaluating an orthodontic aligner"; and Pat. No. US 9,655,693 B2 to Li et al., titled "Multilayer dental appliances and related methods and systems".

### OVERVIEW

The present inventors have recognized, among other things, that problems to be solved in the planning and performance of orthodontic aligner tray systems involve the monitoring and tracking of progress of tooth movement throughout the procedure and performance of the treatment plan. More specifically, the present inventors have recognized the difficulty in obtaining measurements from teeth moved by orthodontic aligner trays. In examples, measuring of the teeth can involve the patient having to undergo a scan of the currently positioned teeth, have the scan digitized into a three-dimensional model, and then comparing the current 3D model to a model generated based on scans of the originally positioned teeth before treatment commenced. Such a process is time consuming and requires allocation of various resources, such as transportation to an orthodontic office location and the use of expensive scanning equipment. As such, mid-treatment measurements are rarely performed in practice. Furthermore, such measurements are only taken when it becomes abundantly clear from a visual inspection by the orthodontist that the treatment plan appears to be off-track, or the patient notices an issue with a specific orthodontic aligner tray. That is, measurements or intra-procedure feedback is not typically obtained to verify that the treatment plan is on-track. Thus, the present inventors have recognized that traditional orthodontic aligner tray approaches lack effective feedback on the amount of tooth movement actually achieved by each orthodontic aligner tray. Furthermore, the present inventors have recognized that traditional orthodontic aligner tray approaches lack the ability to provide feedback as to how the teeth are progressing so al to allow for dynamic planning for subsequent stages of aligner trays.

Furthermore, the present inventors have recognized that orthodontic aligner trays are produced in sets encompassing all or many of the alignment trays from the first tray to the last tray or a tray somewhere in the middle of the treatment plan. As such, if it is determined that a planned tray in the middle of a treatment plan is not performing as anticipated, it can result in new trays being produced and used to continue the treatment plan, thereby resulting in the previously produced trays being obsolete. Thus, the present inventors have recognized that making mid-course corrections to treatment plans can be expensive and wasteful. Furthermore, mid-course corrections can add time to the overall treatment timeline, particularly if the need for the correction is not detected early. Interruptions in the treatment plan can sometimes result in regression of the treatment plan.

The present subject matter can provide solutions to these and other problems, such as by providing systems and methods for obtaining feedback in the performance of orthodontic aligner trays. The systems and methods of the present disclosure can involve obtaining feedback related to the performance of an orthodontic aligner trays in moving teeth from a first position to a second position. In examples, the feedback can be obtained directly from the orthodontic aligner trays. The feedback can be obtained from the orthodontic aligner tray while the patient is wearing the orthodontic aligner tray or with the orthodontic aligner tray removed from the mouth of the patient. The feedback can be obtained after the orthodontic aligner tray has been worn for a period of time, such as at the end of its expected life at the end of a stage in the treatment plan. In examples, photographic images of the orthodontic aligner trays, whether being worn or not, can be used to obtain the feedback. The orthodontic aligner trays can include landmarks that can be recognized in photographic images. Changes in the position of the landmarks can be used to determine how much movement that the teeth have undergone. Thus, before and after images of the orthodontic aligner trays can be compared to evaluate tooth movements. In examples, the landmarks can comprise patterns of indicia produced on the orthodontic aligner trays, such as dots, squares, stars and the like. The indicia can be printed on the aligners or formed into the material of the orthodontic aligner trays. The indicia can maintain the overall appearance and transparency of the orthodontic aligner trays so that the landmarks do not adversely affect the aesthetic appeal of the orthodontic aligner trays. In additional examples, the landmarks can comprise characteristics of the material of the orthodontic aligner trays, such as light conducting or refracting capabilities that change as the material is subject to stress. In examples, the landmarks can comprise overlapping lenses, e.g., those having Moiré Patterns printed thereon, the relative position between the lenses changing as the underlying orthodontic aligner tray changes shape. In examples, the shape of the orthodontic aligner tray can be tracked with a sensor embedded in or attached to the orthodontic aligner tray. The landmarks can be positioned over the entire orthodontic aligner tray or at specific landmark locations where expected changes in the shape of the orthodontic aligner tray is expected to occur or where changes are desired to occur to effectuate the desired tooth movement.

In an example, an orthodontic aligner tray can comprise a shell having a plurality of tooth receiving cavities shaped to apply a repositioning force to teeth of a patient, and a plurality of landmarks present on the shell, wherein the plurality of landmarks are configured to be detected to determine a shape of the shell.

In an example, a method of tracking movement of teeth from application of a first orthodontic aligner tray can comprise designing the first orthodontic aligner tray to have a plurality of landmarks disposed at a first landmark position within a three-dimensional coordinate system for the first orthodontic aligner tray, receiving electronic files of the first orthodontic aligner tray after being applied to teeth so that the first orthodontic aligner tray is deformed to move at least some of the plurality of landmarks into a second landmark position, determining changes in locations of the plurality of landmarks in the three-dimensional coordinate system from the first landmark position to the second landmark position from the electronic files, and outputting an indication of the changes.

In an example, a method for tracking progress of orthodontic treatment using an aligner tray can comprise providing an aligner tray having a plurality of markers disposed thereon, positioning the aligner tray on teeth of a patient, capturing image data of the aligner tray, analyzing the image data to determine positions of the plurality of markers, comparing the determined positions of the plurality of markers with expected positions based on a treatment plan, determining whether the teeth are moving according to the treatment plan based on the comparison, and revising the treatment plan when the comparison indicates that the teeth are not moving according to the treatment plan.

### BRIEF DESCRIPTION OF THE DRAWINGS

FIG. 1 shows a block diagram illustrating examples of fabricating and applying orthodontic aligner trays for orthodontic alignment procedures.
FIG. 2 is a side view of a skull showing a maxillary arch and a mandibular arch of teeth of a patient.
FIG. 3A is a perspective view of a digital model of the maxillary arch and the mandibular arch of the teeth of FIG. 2.
FIG. 3B is a top plan view of a digital model of the mandibular arch of FIG. 3A.
FIG. 4 is a top plan view of an orthodontic aligner tray prepared from the digital model of FIG. 3B.
FIG. 5 is a side cross-sectional view of an orthodontic aligner tray having a tooth disposed therein showing force bumps engaged with the tooth.
FIG. 6A is a top cross-sectional view of a row of teeth disposed between walls of an orthodontic aligner tray at the beginning of application of the orthodontic aligner tray.
FIG. 6B is a top cross-sectional view of the row of teeth disposed between walls of the orthodontic aligner tray of FIG. 6A at the end of application of the orthodontic aligner tray.
FIG. 7 is a block diagram illustrating examples of tracking changes of teeth positions from the application of orthodontic aligners using the tracking devices, systems and methods of the present disclosure.
FIG. 8A is a schematic view of landmarks that can be applied to orthodontic aligner trays to facilitate tracking.
FIG. 8B is a perspective view of an orthodontic aligner tray having the landmarks of FIG. 8A.
FIG. 8C is a perspective view of the orthodontic aligner tray of FIG. 8B positioned over teeth.
FIG. 9 is a schematic illustration of a set of landmarks disposed in a three-dimensional coordinate system.
FIG. 10 is a schematic illustration of an orthodontic aligner tray having light diffraction capabilities that can be used to perform tooth position tracking according to the present disclosure.
FIG. 11A is a schematic illustration of a tooth having an orthodontic aligner tray positioned around the tooth to show stress applied to the orthodontic aligner tray by the tooth with the orthodontic aligner tray and the tooth in a first relative position.
FIG. 11B is a schematic illustration of a tooth having an orthodontic aligner tray positioned around the tooth to show stress applied to the orthodontic aligner tray by the tooth with the orthodontic aligner tray and the tooth in a second relative position.
FIG. 12A is a perspective view of an orthodontic aligner tray having contour lines that can be used to perform tooth position tracking.
FIG. 12B is an example schematic view of slits formed into the orthodontic aligner tray of FIG. 12A to match the contour lines.
FIG.13A is a schematic illustration of an orthodontic aligner tray surface having a first pattern of lines overlayed with a lens having a second pattern of lines to form windows having a shape.
FIG. 13B is a schematic illustration of the orthodontic aligner tray surface and lens of FIG. 13A with relative translation therebetween to show changes in the windows.
FIG. 14 is a perspective view of an orthodontic aligner tray having a sensor that can be used to perform tooth position tracking.
FIG. 15 is a schematic illustration of a system for manufacturing an orthodontic aligner tray and determining changes in shape of the orthodontic aligner tray.

### DETAILED DESCRIPTION

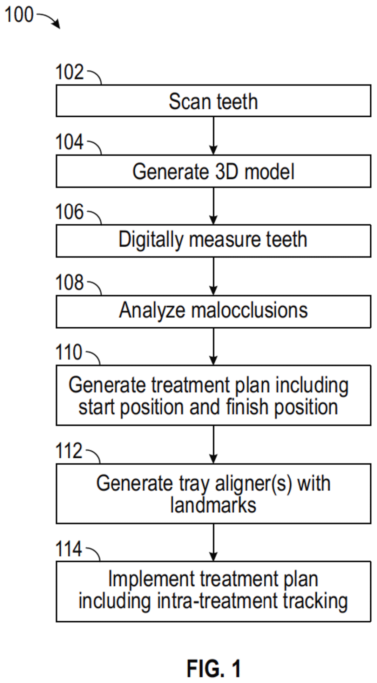

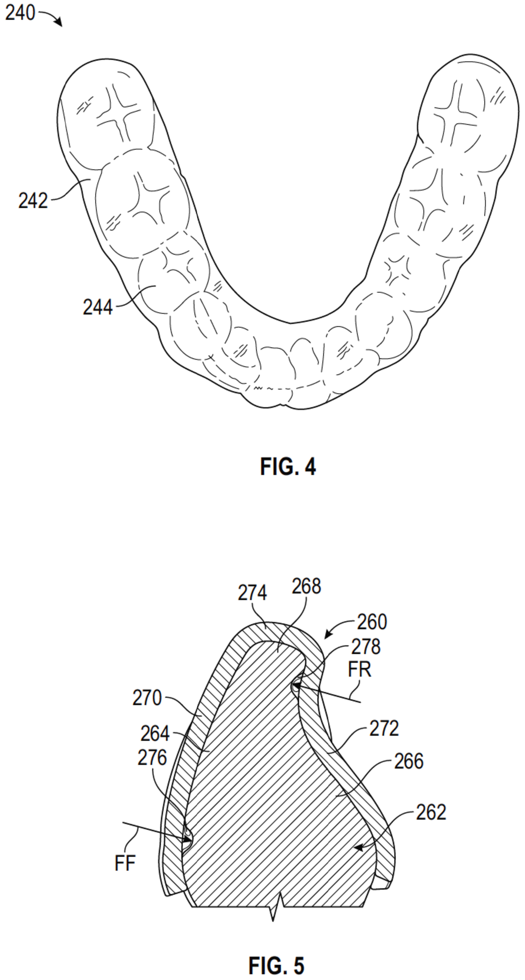

FIG. 1 shows block a diagram illustrating operations of method 100 for fabricating and applying orthodontic aligner trays. FIG. 1 describes operations for generating orthodontic aligner trays that can include landmarks as described herein. As discussed with reference to FIG. 5 through FIG. 15, the landmarks can be used to track changes in the shape of the orthodontic aligner tray, which can be used to determine changes in the position of teeth from the beginning of the application of an orthodontic aligner tray to the end of the application of the orthodontic aligner tray. As such, the progress that each orthodontic aligner tray achieves in moving the teeth according to a treatment plan can be determined, thereby more efficiently permitting manufacture of the subsequent orthodontic aligner tray.

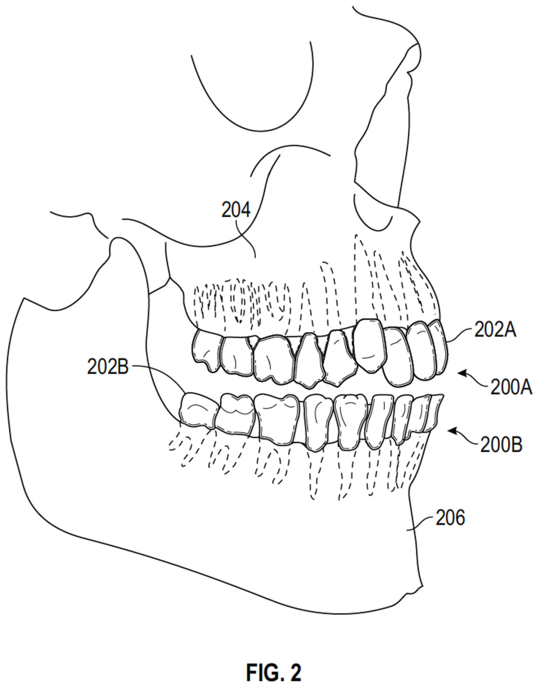

At operation 102, teeth 202A and teeth 202B (FIG. 2) of a patient can be scanned into a digital file, e.g., using a scanning software system. In examples, in-mouth scanning can be performed using small hand-held scanners. In examples, an intraoral scanning device as described in US 9,101,329 B2 to Ertl, titled "Method for Determining the Position of an Intraoral Measuring Device" can be used. In examples, impressions of teeth can be scanned using freestanding scanning systems, such as 3Shape R700, by 3shape A/S, of Copenhagen, Denmark (hereinafter referred to as 3shape). In additional examples, the OrthoPlex or Orthophos S orthodontic digital modeling solution available from Dentsply Sirona Inc. of Charlotte, North Carolina can be used. The scanner and its associated software can create a three-dimensional (3D) digital model that can be accessed by a computer or the like, and can be stored as an STL file. An STL file is a conventional format often used by stereolithography software to generate information needed to produce 3D models on stereolithography machines. While it is not necessary to use this file format, the format is useful to facilitate the use of fabricating orthodontic aligner trays using stereolithography or other rapid manufacturing processes.

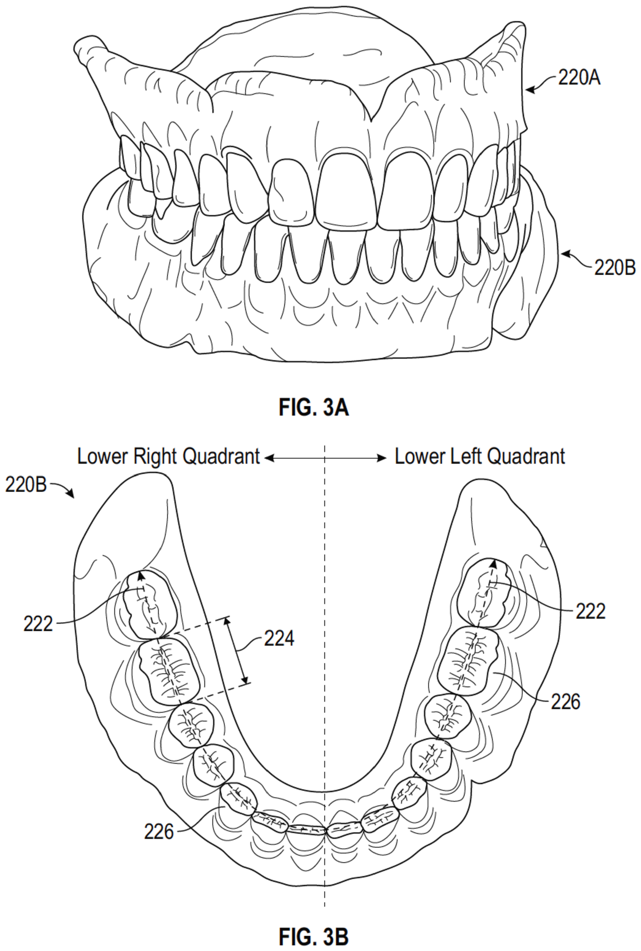

At operation 104, a 3D model of the teeth can be generated based on the scanned digital files. For example, maxillary arch model 220A and mandibular arch model 220B of FIG. 3A can be generated. The digital model can include the shape of each individual tooth of teeth 202A and teeth 202B as well as the placement of each tooth within maxilla 204 and mandible 206 (FIG. 2). As such, maxillary arch model 220A and mandibular arch model 220B can show the relative position and alignment, or misalignment, of each tooth of teeth 202A and each tooth of teeth 202B. In examples, the files can comprise Computer-Aided Design (CAD) files or the like, such as DWG files, DICOM files and others.

At operation 106, tooth width and arch width of the teeth can be measured digitally, e.g., using Ortho Analyzer software. Digital measurements can be taken of maxillary arch model 220A and mandibular arch model 220B. Computer-aided planning tools can be used to determine a complete three-dimensional coordinate set for all of teeth 202A and teeth 202B. For example, measurements of mesio-distal width 224 (FIG. 3B) of each tooth relative to mesio-distal direction 222 can be determined.

At operation 108, the positions of teeth 202A and teeth 202B (FIG. 2) can be analyzed to determine misalignment and malocclusion. For example, the amount of rotation of mesio-distal width 224 of each tooth relative to mesio-distal direction 222 can be determined.

At operation 110, a treatment plan can be generated. The treatment plan can be generated using software, e.g., Ortho Analyzer Next Generation software from 3shape or CEREC Ortho SW 2.0 software from Dentsply Sirona. The treatment plan can include the planning of the shape and size of a plurality of orthodontic aligner trays to be used to move the teeth from the starting position of the 3D model to a final, aligned position. The treatment plan can include the performance of one or more Inter Proximal Reductions (IPRs) at selected locations along the treatment plan, e.g., to be performed between particular orthodontic aligner tray iterations.

At operation 112, one or a plurality of orthodontic aligner trays or aligner trays can be fabricated to fit the treatment plan. In one exemplary embodiment, the aligner trays can be thermo-formed over a physical overlay model using, for example, an Essix vacuum forming machine and Essix Ace plastic thermoforming sheets. Both the sheets and the thermoforming machine are commercially available from Dentsply Sirona, Inc. In examples, the thermoforming process can comprise conventional and known processes in the dental art. Using a physical overlay model to produce the aligner trays can result in a clear pathway of space from the teeth positions before patient treatment to the teeth positions after treatment, created to allow for tooth movement. In other examples, the aligner trays can be fabricated using other manufacturing methods, such as additive manufacturing processes and three-dimensional printing processes. As discussed, the pathway of space for the teeth positions can include the performance of one or more IPR procedures. The orthodontic aligner trays can be manufactured to include landmarks or other features or devices, over the entire orthodontic aligner tray or at selected landmark locations, that can be used to track change in position of teeth from the beginning of the application of the orthodontic aligner tray to the end of the application of the orthodontic aligner tray, as discussed herein. The landmarks can be added to the as-produced orthodontic aligner trays, or can be added during the manufacturing of the orthodontic aligner trays.

The total number of aligners trays in a set to complete a treatment plan can vary from case to case. In conventional systems, the entire set of aligners is manufactured at the beginning for providing to the patient. As mentioned, this can result in some of the aligners not being used if issues arise in the performance of the treatment plan. However, with the present disclosure, less than the entire set of aligners can be manufactured. Due to the ability to track the movements of teeth induced by each aligner, such as by using the landmarks described herein, each aligner tray after the first aligner tray can be manufactured based on the performance of the application of the previous aligner tray, rather than based on expected performance as outlined in the treatment plan.

At operation 114, the treatment plan developed at operation 110 can be implemented. Implementation of the treatment plan can include performing intra-treatment tracking of the teeth during one or more stages, as well as refining of the treatment plan to account for performance of a previously used orthodontic aligner tray. Specifically, as described with reference to FIG. 5, operation 114 can include any or all of the operations described with reference to operation 310 through operation 328.

FIG. 2 is a side view of a skull including maxillary tooth arch 200A and mandibular tooth arch 200B. Maxillary tooth arch 200A can comprise teeth 202A extending from maxilla 204. Mandibular tooth arch 200B can comprise teeth 202B extending from mandible 206. Maxillary tooth arch 200A can comprise teeth 202A, such as molars, premolars, canines and incisors. Mandibular tooth arch 200B can comprise teeth 202B, such as molars, premolars, canines and incisors. In examples, some or all of teeth 202A can be situated in maxilla 204 in a misaligned manner, and/or some or all of teeth 202B can be situated in mandible 206 in a misaligned manner.

FIG. 3A is a perspective view of a digital model comprising maxillary arch model 220A and mandibular arch model 220B. Maxillary arch model 220A and mandibular arch model 220B can comprise nearly identical digital reproductions of maxillary tooth arch 200A and mandibular tooth arch 200B, respectively, including the alignment of teeth 202A and teeth 202B. However, multiple digital models can be produced to include gradual changing of the position of some teeth to correct the malocclusions, as discussed herein, to produce a series of dental alignment trays. The original 3D model that is an accurate representation of the current state of teeth 202A and teeth 202B can be modified by the orthodontic planning software at operation 110 to generate one or more modified 3D models corresponding to each staging of an orthodontic aligner tray. Thus, the digital models can form the basis for the production of aligner tray stagings, such as via the aforementioned overlay process.

FIG. 3B is a top plan view of mandibular arch model 220B of mandibular tooth arch 200B of FIG. 2. Each tooth of teeth 202B, as represented by model teeth 226, can have a mesial side that faces forward or the tooth in front of it and a distal side that faces backward or the tooth behind it. The buccal side of the tooth extends between the mesial side and the distal side and faces toward the cheek. The lingual side of the tooth extends between the mesial side and the distal side and faces toward the tongue. It is desirable from an aesthetics standpoint and from a functional standpoint to have each of teeth 202A and teeth 202B align such that the mesial sides and the distal sides are aligned. The term "mesio-distal width" of a tooth refers to the width or diameter of a tooth form as measured across the mesio-distal direction 222 of the dentulous arch form.

However, for a multitude of reasons, teeth 202B can grow into misaligned positions where in the mesial sides and the distal sides of the teeth are not suitably aligned along the mesio-distal direction 222. The malocclusions between teeth can have varying levels of misalignment that can dictate the treatment plan for the generation of aligner trays. In particular, the amount of rotation of one tooth relative to an adjacent tooth can determine if or when an interproximal reduction is in order.

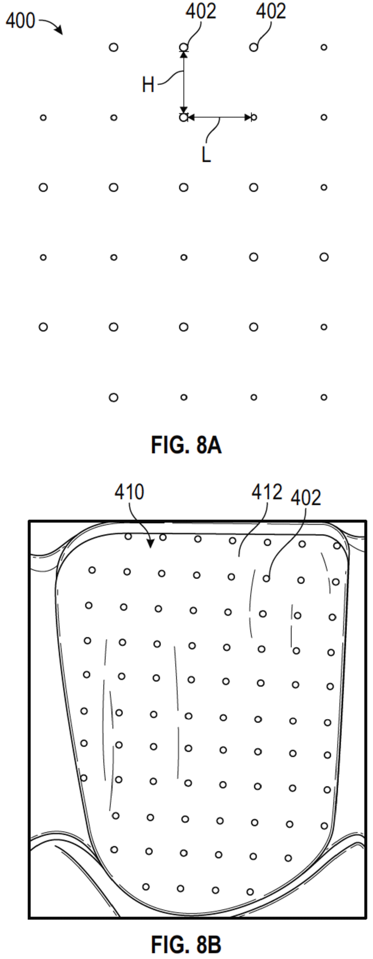

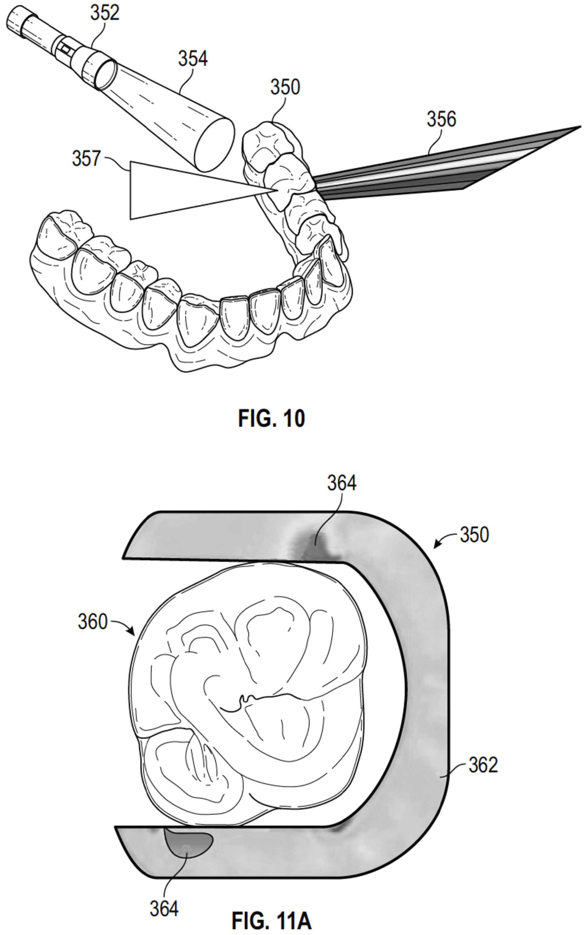

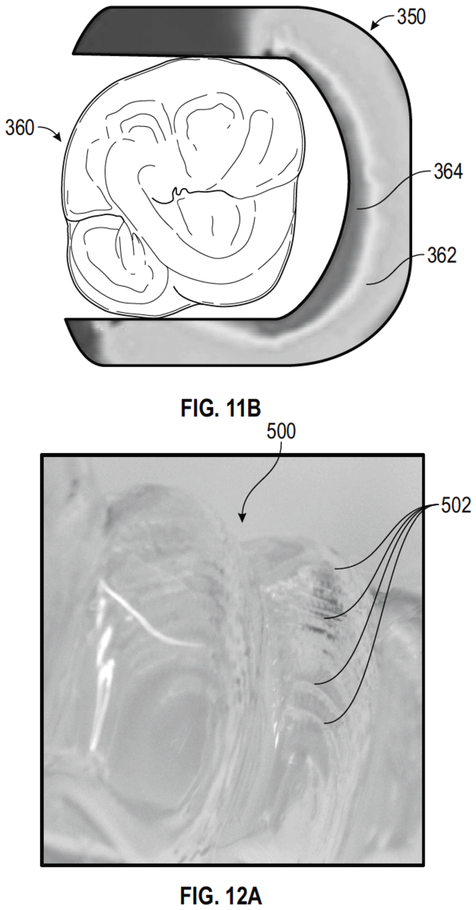

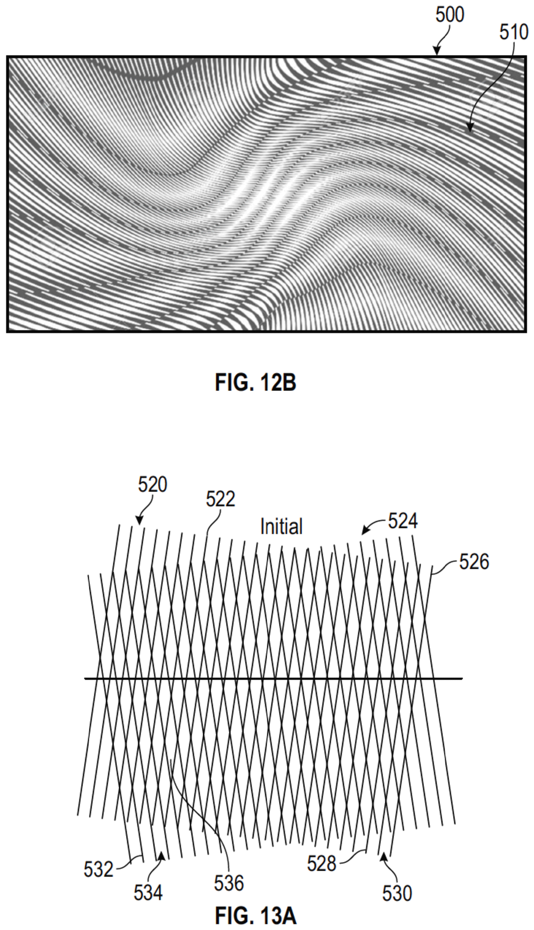

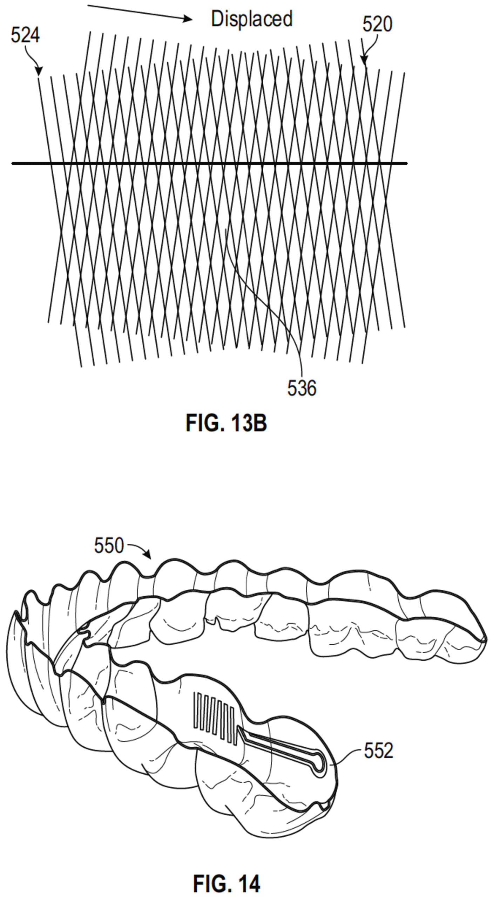

FIG. 4 is a top plan view of aligner tray 240 prepared from mandibular arch model 220B of FIG. 3B. Aligner tray 240 can include base 242 and pockets 244. Base 242 can comprise an arcuate trough shaped to fit around mandibular tooth arch 200B. Each pocket 244 can form a compartment for receiving one of teeth 202B. One, some or all of pockets 244 can be configured to apply pressure to a tooth of teeth 202B to gradually move the tooth into a different position according to the treatment plan. As discussed herein, a series of aligner trays similar to aligner tray 240 can be generated wherein the shape and/or size of one or more of pockets 244 changes from one staging to the next to gradually move the teeth to a desired position. The series of aligner trays can be designed to include IPR procedures being performed between one or more stagings. In examples, aligner trays can be designed and manufactured according to those described in US. Pat. No. 8,123,519 to Schultz, titled "Method and System for Personalized Orthodontic Treatment," the entire contents of which are incorporated herein in their entirety by this reference. Furthermore, as discussed herein, aligner tray 240 can be manufactured or otherwise produced to include one or more of the landmarks of FIG. 8A, the light diffraction capabilities of FIG. 10 through FIG. 11B, the contour lines of FIG. 12A and FIG. 12B, the Moiré Pattern lens of FIG. 13A and FIG. 13B, and the sensor of FIG. 14.

FIG. 5 is a side cross-sectional view of tooth 260 surrounded on front, top and rear sides by orthodontic aligner tray 262. Tooth 260 can include mesial side 264, buccal side 266 and apex 268. Orthodontic aligner tray 262 can comprise front wall 270, rear wall 272 and top wall 274. Orthodontic aligner tray 262 can additionally comprise front force bump 276 and rear force bump 278. As discussed herein, orthodontic aligner tray 262 can be configured to apply forces to tooth 260 to move tooth 260 into a desired position. In particular, orthodontic aligner tray 262 can have a shape in a relaxed position when not worn over teeth. In examples, the starting relaxed shape can correspond to a desired final position of tooth 260 resulting from application of orthodontic aligner tray 262 for that stage of the treatment plan. In other examples, the starting relaxed shape can be different than the desired final position of tooth 260 resulting from orthodontic aligner tray 262 for that stage of the treatment plan and can deform to the desired final position of tooth 260. However, other starting relaxed shapes can also be used. Thus, the starting relaxed shape of orthodontic aligner tray 262 can be different than the final stressed shape of orthodontic aligner tray 262 after being applied to tooth 260 for that stage of the treatment plan. Thus, when orthodontic aligner tray 262 is positioned over tooth 260 front wall 270 and rear wall 272 can apply force to tooth 260. Additionally, front force bump 276 can apply front force FF to mesial side 264 of tooth 260 and rear force bump 278 can apply rear force FR to buccal side 266 of tooth 260. Note, FIG. 5 schematically illustrates front force bump 276 and rear force bump 278 extending into the boundary of tooth 260. In actual use, front force bump 276 would push against the surface of tooth 260 and space being present between front wall 270 and buccal side 266, and rear force bump 278 would push against the surface of tooth 260 and space being present between rear wall 272 and buccal side 266.

In the illustrated example, front wall 270 can be engaged generally flush with buccal side 266 and rear wall 272 can be engaged generally flush with buccal side 266. However, depending on the particular tooth shape and the particular way the tooth is intended to be moved, portions of front wall 270 and rear wall 272 can be out of contact with tooth 260. Thus, over time portions of orthodontic aligner tray 262 can move into or out of contact with tooth 260 as tooth 260 is physically displaced relative to bone matter in which it is disposed, e.g., maxilla 204 or mandible 206. Thus, the final relaxed shape of orthodontic aligner tray 262 at the end of applying orthodontic aligner tray 262 for that state of a treatment plan is different than the starting relaxed shape of orthodontic aligner tray 262 at the beginning of the application of orthodontic aligner tray 262. Likewise, the final stressed shape of orthodontic aligner tray 262, as worn by the patient, at the end of applying orthodontic aligner tray 262 is different than the starting relaxed shape of orthodontic aligner tray 262 at the beginning of the application of orthodontic aligner tray 262 or the starting shape of orthodontic aligner tray as worn by the patient at the beginning of applying orthodontic aligner tray.

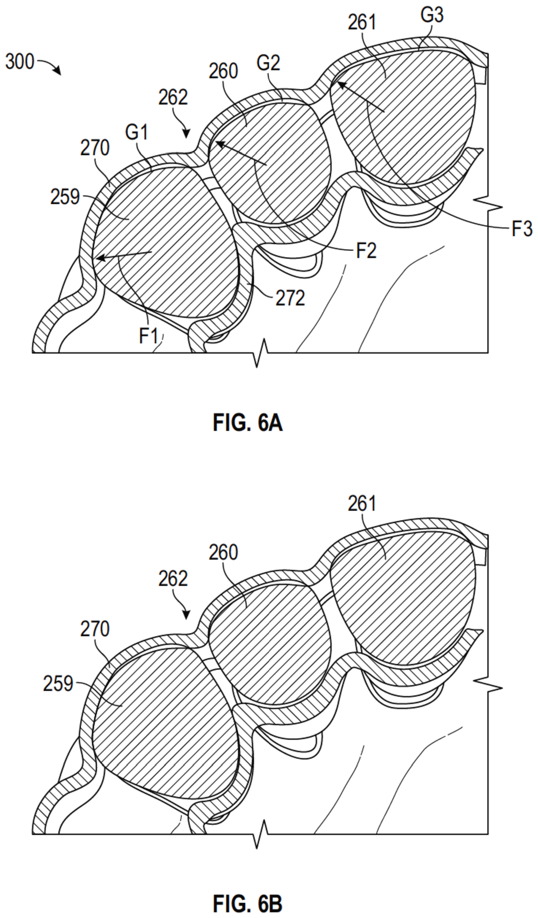

FIG. 6A is a top cross-sectional view of tooth 260 disposed between front wall 270 and rear wall 272 of orthodontic aligner tray 262 at the beginning of application of the orthodontic aligner tray 262 at a stage of a treatment plan. Tooth 260 is positioned between tooth 259 and tooth 261. FIG. 6A schematically shows tooth 259 through tooth 261 within orthodontic aligner tray 262 including gaps therebetween. However, in practice, tooth 260 could be touching one or both of tooth 259 and tooth 261.

When orthodontic aligner tray 262 is new, e.g., before being applied to teeth, it can have a particular relaxed state where no stress is being applied to the material of orthodontic aligner tray 262. However, when orthodontic aligner tray 262 is positioned around tooth 259 through tooth 261, orthodontic aligner tray 262 can become distorted to fit around the teeth, which have not yet moved into the desired final position of the shape of orthodontic aligner tray 262. As such, gaps can be present between tooth 259 through tooth 261 and front wall 270 and rear wall 272. For example, gap G1 can be located between tooth 259 and front wall 270, gap G2 can be located between tooth 260 and front wall 270 and gap G3 can be located between tooth 260 and front wall 270. Correspondingly, front wall 270 can engage with tooth 259 through tooth 261 at pressure points that result in orthodontic aligner tray 262 asserting force against tooth 259 through tooth 261. For example, front wall 270 can assert force F1 at a corner of tooth 259, front wall 270 can assert force F2 at a corner of tooth 260 and front wall 270 can assert force F3 at a corner of tooth 261. Thus, over time, tooth 259 through tooth 261 can be rotated by force F1 - force F3, respectively, to close gap G1 - gap G3, respectively. The forces can induce stress within the material of orthodontic aligner tray 262 that can result in a deformation of orthodontic aligner tray 262 both when applied to tooth 259 through tooth 261 and when removed from tooth 259 through tooth 261 due to, for example, stress relaxation.

FIG. 6B is a top cross-sectional view of tooth 260 disposed between front wall 270 and rear wall 272 of orthodontic aligner tray 262 at the end of application of the orthodontic aligner tray 262 at the stage of FIG. 6A of the treatment plan. FIG. 6B shows gap g1 - gap G3 being closed such that front wall 270 is generally flush with tooth 259 through tooth 261. As a result of reaction forces from tooth 259 through tooth 261 being applied to orthodontic aligner tray 262, the material of orthodontic aligner tray 262 can change as the positions of tooth 259 through tooth 261 change. As discussed herein, the shape of orthodontic aligner tray 262 after being applied to tooth 259 through tooth 261 for a period of time while being worn over tooth 259 through tooth 261 can provide feedback as to the movement of the teeth induced by orthodontic aligner tray 262.

Furthermore, if orthodontic aligner tray 262 is removed from tooth 259 through tooth 261, orthodontic aligner tray 262 will relax. However, the effects of being exposed to force F1 - force F3 over a prolonged period of time, e.g., multiple weeks, can result in the material of orthodontic aligner tray 262 retaining some of the deformation when removed from tooth 259 through tooth 261. For example, orthodontic aligner tray 262 can be made from a plastic or polymer that is viscoelastic, such as polyethylene terephthalate (PET) or PETG (polyethylene terephthalate glycol), meaning that the material experiences fatigue loss; for example, because the material is not a perfect spring that returns to its original shape when forces applied thereto are removed it retains some of the previously applied deformation. Thus, the at rest shape of orthodontic aligner tray 262 after being applied to tooth 259 through tooth 261 for a period of time and then being removed from the teeth can still provide feedback as to the movement of the teeth induced by orthodontic aligner tray 262. Mathematical models can be used to determine the stress relaxation of orthodontic aligner tray 262 after being applied to teeth that would result in an intermediate shape between that of FIG. 6A and that of FIG. 6B, e.g., where gap G1 - gap G3 might be smaller than shown in FIG. 6A.

With the present disclosure, the shape of orthodontic aligner tray 262 can be tracked, measured or determined from the starting shape of FIG. 6A to the resulting, ending shape of FIG. 6B in order to track changes in the positions of tooth 259 through tooth 261, such as to determine if tooth 259 through tooth 261 are moving or have moved according to that stage of the treatment plan. That is, changes in the shape of orthodontic aligner tray 262 can serve as a proxy for the changes in position of tooth 259 through tooth 261. Mathematical models can be used to determine the change in shape of orthodontic aligner tray 262 after being applied to teeth that would result in the desired amount of tooth movement according to the treatment plan. Thus, as described below, landmarks, fiducial markers, material property markers and the like can be added to orthodontic aligner tray 262 to assist in visualizing, e.g., digitizing, the shape of orthodontic aligner tray.

In examples, the starting shape can comprise orthodontic aligner tray 262 as designed, in an unstressed or undeformed state, e.g., not applied to teeth. In examples, the starting shape can comprise orthodontic aligner tray 262 as applied to teeth in a stressed or deformed shape at the very beginning of the treatment stage. In examples, the starting shape can be based on computer-aided design (CAD) files of orthodontic aligner tray 262 or photographic images of orthodontic aligner tray 262 removed from tooth 259 through tooth 261 before being worn for a period of time. In examples, the starting shape can be based on photographic images of orthodontic aligner tray 262 worn on tooth 259 through tooth 261 before being worn for a period of time.

In examples, the ending shape can comprise orthodontic aligner tray 262 as designed, in an unstressed or undeformed state, e.g., not applied to teeth, after being applied to teeth for a period of time. In examples, the ending shape can comprise orthodontic aligner tray 262 in a stressed or deformed state as applied to teeth at the very end of the treatment stage after being worn for a period of time. In examples, the ending shape of orthodontic aligner tray 262 can be based on images of orthodontic aligner tray 262 while applied to tooth 259 through tooth 261 after being worn for a period of time. In examples, the ending shape can be based on photographic images of orthodontic aligner tray 262 removed from tooth 259 through tooth 261 after being worn for a period of time.

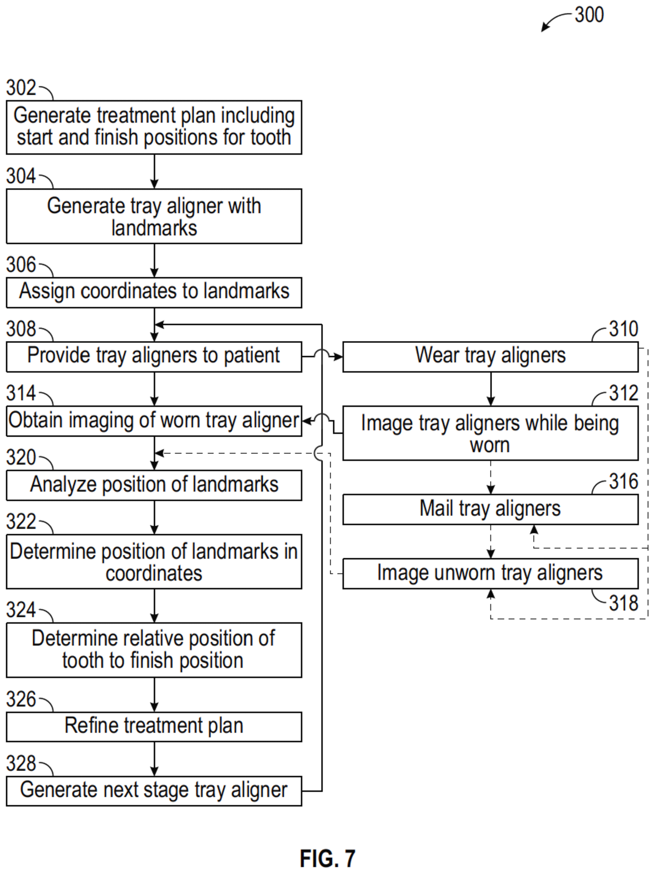

FIG. 7 is a block diagram illustrating examples of method 300 for tracking changes of teeth positions from the application of orthodontic aligners using the tracking devices, systems and methods of the present disclosure. Though discussed with reference to FIG. 1 through FIG. 6B and FIG. 8A - FIG. 15 and particular examples of methods and systems for tracking changes in tooth position, method 300 can encompass the use of any tracking system and method consistent with the methods and systems described herein. Method 300 can additionally include fewer or greater operations other than operation 302 to operation 328. Additionally, in other examples, operation 302 through operation 328 can be performed in other sequences.

At operation 302, a treatment plan can be generated to include start and finish positions for the teeth. The treatment plan can additionally include start and finish positions for each of the aligner trays in order to move the teeth from the overall start position to the overall finish position. However, as discussed herein, the treatment plan can include only the start and finish positions for the initial or first aligner tray, with the start and finish positions for the subsequent aligner trays, e.g., the second aligner tray, being later developed based on performance of the first aligner tray. In examples, the treatment plan can include planned shapes for orthodontic aligner trays to move the teeth from the start position to the finish position. The planned shapes can include the shape of each orthodontic aligner tray before being applied to teeth and after being applied to teeth for a period of time to affect the desired tooth movement. In examples, operation 302 can include all or some of the elements of operation 102 through operation 110 of FIG. 1.

At operation 304, the first aligner tray can be generated or manufactured. The first aligner tray can include landmarks, such as those described with reference to FIG. 8A through FIG. 9. For example, the landmarks can comprise an array of small shapes, such as dots, triangles, rings, rectangle, stars and the like scattered over the surface of the aligner tray. In examples, the landmarks can comprise light diffraction capabilities, such as those within designated landmark areas or locations on the aligner tray, that can identify stress within the aligner tray, as described with reference to FIG. 10 - FIG. 11B. In examples, the landmarks can comprise ridges or contour lines on the surface of the aligner tray, as described with reference to FIG. 12A and FIG. 12B. In examples, the landmarks can comprise Moiré Pattern lenses, as described with reference to FIG. 13A and FIG. 13B. In examples, the landmarks can comprise one or more positions delineated by one or more sensors, as described with reference to FIG. 14. In examples, the landmarks can be on the exterior surface of the aligner tray (e.g., tooth-facing surface), embedded in the material of the aligner tray, or on the interior (e.g., mouth-facing surface) of the aligner tray. In examples, the landmarks can be applied with uniform density over substantially the entire aligner tray, or can be positioned only in selected landmark locations where it is desirable to track shape changes specifically related to tooth movements.

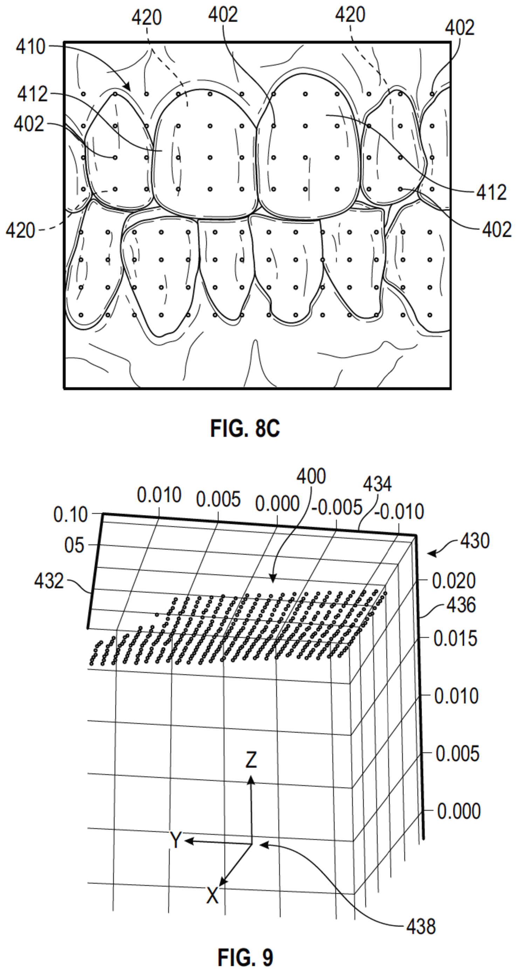

At operation 306, the positions of the landmarks can be pre-registered to a three-dimensional coordinate system, such as that shown in FIG. 9. In examples, any of the landmarks can comprise an origin for the 3D coordinate system. However, in examples, the landmarks need not be registered to any coordinate system, and changes in the relative position of each landmark can be determined before and after an aligner tray is worn for a period of time. In such cases, any point on the aligner tray can be used as reference, e.g. an origin, to determine the before and after shape of the aligner tray. In examples, the landmarks can be arranged in a symmetric pattern, e.g., equidistant from adjacent landmarks, at the start. However, in examples, the landmarks can be in any position, e.g., randomly distributed, at the start. In examples, the landmarks can be any perceivable property, pattern or shape in or on the aligner tray, such as ripples, waves, slits, Moire patterns and the like, throughout the whole aligner tray or is designated landmark locations correlated to, for example, high stress areas associated with applied forces from teeth.

At operation 308, the aligner trays can be provided to a patient. For example, the aligner trays can be shipped from a manufacturing facility to the patient, or the aligner trays can be sent to a dental professional and handed over to the patient during an office visit. In additional examples, manufacturing specifications can be provided to a patient electronically for producing at home, such as with a 3D printer or additive manufacturing machine.

At operation 310, the patient can wear the aligner tray. The patient can wear the first aligner tray for the prescribed amount of time, e.g., a particular number of days, weeks or months determined by the dental professional. In examples, aligner trays are used for one to two weeks. In examples, the amount of time is sufficient to allow teeth to be moved by the aligner tray and/or to allow stress deformation of the aligner tray to occur.

At operation 312, the patient can take images of the aligner trays, e.g., upper and lower aligner trays. At operation 312 the patient can take images of the aligner trays while wearing the aligner tray over their teeth. The images can include timestamps of when the images were obtained. For example, the patient can use a digital camera, such as that of a smartphone or tablet computer, to obtain images of the aligner tray in their own mount. In examples, the patient can use the flash of the camera or another light source to illuminate the aligner trays to, for example, provide better lighting and/or to trigger or enhance particular properties of the aligner trays, e.g., invisible ink or refractive capabilities. In examples, the patient can use a handheld 3D scanner that can be inserted into the mouth to obtain images. In examples, images can be obtained from combinations of cameras and 3D scanners. Multiple images can be taken at different angles and positioned of the camera relative to the mouth. In examples, the patient can smile or otherwise open their lips to allow visual access to the teeth. In examples, the patient can use a dental retractor to facilitate opening of the lips to provide wider views of the teeth. In examples, the patient can take the photographic images themselves or can have another person assist in taking the photographic images. Thus, the patient can generate or obtain the photographic images from their own home or any location convenient for them.

In additional examples, the patient can travel to a dental or orthodontic office to have images of the teeth generated. In examples, a dentist or orthodontics or other dental professional can take photographic images of the teeth wearing the aligner trays. However, orthodontic professionals can additionally use a handheld 3D scanner to obtain the images.

In additional examples, the patient can additionally or alternatively take images of the used aligner trays not being worn. The images can include timestamps of when the images were obtained. Furthermore, the patient can record a time period between when the aligner trays were removed from being worn on the teeth and when the images were taken.

At operation 314 the images from the patient can be transmitted to a facility for analysis. For example, the images obtained by the patient can be transmitted via wireless communication from a smartphone or tablet computer using, for example, the Internet, wireless communication signals, text message, email or the like. In examples, a dedicated application can be installed on the smartphone or tablet computer to take, store and transmit the images directly to an analysis facility without the patient having to specifically prepare a separate correspondence.

Operation 316 and operation 318 can comprise optional operations, as shown in in dotted lines. At operation 316, unworn (e.g., trays not in a mouth) and used (e.g., worn for a period of time) aligner trays can be mailed or otherwise transmitted to the facility for taking pictures of the aligner trays after they have been used. Operation 316 can be performed as an alternative to the patient generating images on their own at a location of their preference. In examples operation 316 can be performed in combination with operation 312.

At operation 318, the facility can obtain images of the unworn aligner trays, such as by using photographic equipment or 3D scanners. The facility can receive the unworn and used aligner trays at operation 314.

At operation 320, the landmarks on the aligner trays can be analyzed in combination with any temporal information, such as timestamps, from when the images were taken and any time period between removal and imaging. Either images provided by the patient or images obtained by a dental professional can be analyzed to determine the position of the landmarks. Additionally, or alternatively, the used, e.g., worn to the end of an aligner stage, aligner trays can be scanned with 3D scanners to determine the shape of the aligner tray and the locations of the landmarks on the aligner trays. The analysis can vary depending on when data is captured (worn state, on a model or in the relaxed state). If in relaxed state, a time can be allowed to elapse prior to data acquisition to permit for relaxation. In examples, sufficient relaxation can occur in fifteen minutes. For example, the coordinates of a pattern of landmarks can be determined before and after the aligner tray is used in order to determine how much each landmark has moved, which can then be mathematically interpreted to determine the amount of tooth movement that resulted from the corresponding change in landmark positions.

At operation 322, the position of the landmarks can be determined within a coordinate system. In particular, the landmarks can be analyzed to determine their position within the three-dimensional coordinate system assigned at operation 306 or to determine relative changes in position of one or more landmarks. Changes in the position of one or more landmarks can be determined between the shape of the used aligner tray and the unused aligner tray.

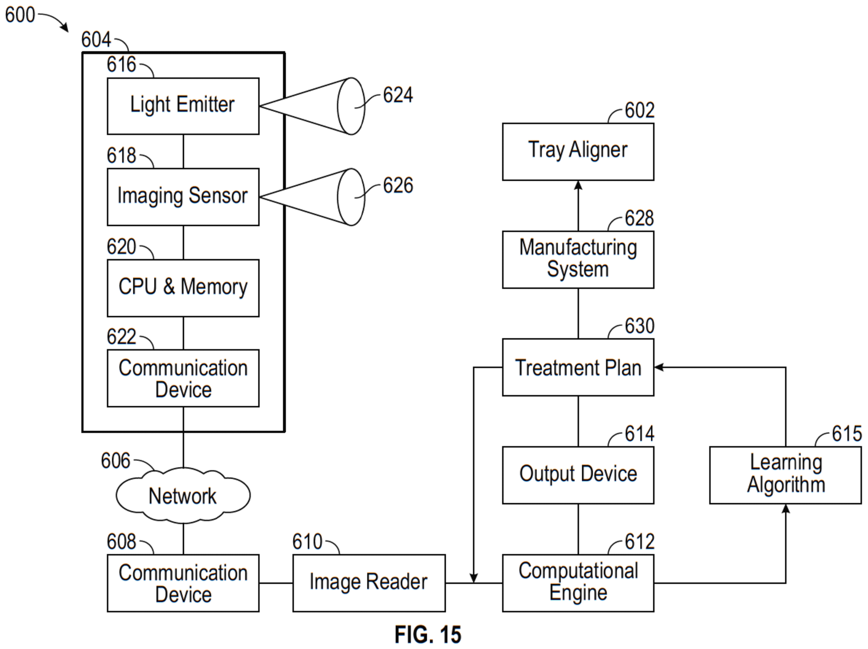

At operation 324, changes in the shape of the aligner tray can be correlated to changes in position of teeth that wore the aligner tray. As discussed herein, the counter forces of the teeth pushing against the aligner tray can induce a shape change of the aligner tray. Mathematical models or simulations can be run to determine the associated tooth movement with the change in shape of the aligner tray. In examples, the aforementioned Ortho Analyzer Next Generation software can be used to determine the changes in tooth position based on changes in the shape of the aligner tray. In examples, system 600 of FIG. 15, including computational engine 612, can be used to determine the changes in tooth position based on changes in the shape of the aligner tray.

At operation 326, the treatment plan can be updated or refined, if necessary. It can be determined if the stage of the aligner tray has moved the teeth according to the treatment plan. If it is determined that the teeth have move according to the plan, the next stage of the aligner trays that was previously planned can be used that picks up the treatment plan from where the just-used aligner tray was planned to have left the teeth. If it is determined that the teeth have not moved according to the plan, a new aligner tray can be fabricated that picks up the treatment plan from where the just-used aligner tray left the teeth not according to the treatment plan at operation 326. As such, a previously planned treatment plan can be continued or updated, or an in-progress, e.g., incomplete, treatment plan can be continued.

At operation 328, the next stage aligner tray can be manufactured. The aligner tray can be manufactured according to any method described herein or a conventional manner, including the addition of landmarks for the new aligner tray and projected positions for the landmarks if the aligner tray was successfully moved the teeth to the desired position of the treatment plan. Additionally, previously manufactured aligner trays can be used if it is determined that the just-used aligner tray moved the teeth according to the treatment plan.

FIG. 8A is a schematic view of landmarks 400 that can be applied to orthodontic aligners, such as aligner tray 240 of FIG. 4, to facilitate tracking of teeth. In the illustrated example, landmarks 400 can comprise a plurality of small circles or dots 402 arranged in a grid format. In examples, the center of each of each of dots 402 can be separated from the center of a neighboring dot by an equal distance, such that heigh H is equal to length L. Thus, the grid can comprise square grid spacing. However, in other examples, dots 402 can be arranged in other patterns, such as a diamond pattern or can be touching so as to provide one or more groupings or agglomerations. Additionally, in other examples, dots 402 can be arranged in asymmetric patterns or not with any pattern at all. In the illustrated examples, dots 402 can have different diameters. However, in examples, each dot can have the same diameter. In examples, each of dots 402 can have a diameter in the range of approximately 5 microns (~0.005 millimeters) to approximately 200 microns (~0.2 millimeters). The density of dots 402 can be sufficient to provide coverage for each tooth, whether arranged in spaced patterns or tight groupings. In examples, the front surface of each tooth can be provided with twelve dots 402. However, the front of each tooth can be provided with a number of dots 402 in the range of approximately four dots 402 to approximately forty dots. The size, number and density of dots 402 can be selected to minimized noticeability and impacts to aesthetic appeal, while still allowing for definitive recognition in digital images.

FIG. 8B is a perspective view of orthodontic aligner tray 410 having landmarks 400 of FIG. 6A. Orthodontic aligner tray 410 can have substrate 412, which can comprise a shell structure, into which dots 402 are integrated. In examples, dots 402 can be painted onto substrate 412. As such, various known biocompatible paints or inks can be used. In examples, parylene paint, and epoxy and urethane coatings can be used. In examples, biocompatible ink can be fabricated using one or more of gelatin, gelatin methacryloyl (GelMA), collagen, alginate, hyaluronic acid, agarose, and fibrin, as well as others. In examples, a transparent material can be added to the aligner tray to form a bump or protrusion. In examples, dots 402 can be burned or burnished into substrate 412, such as to cause a discoloration in the material of the aligner tray or a localized deformation in the shape of the aligner tray, such as dimple or bump. In examples, bumps or depressions can be formed in orthodontic aligner tray 410 as the thermoforming sheets are vacuum formed over the overlay. In examples, dots 402 can be provided equally over the entire surface of orthodontic aligner tray 410, or can be placed only in strategic locations where it is particularly desirable to monitor shape changes, such as in areas of expected high stress.

FIG. 8C is a perspective view of orthodontic aligner tray 410 of FIG. 8B positioned over teeth 420. Landmarks 400 can be positioned over teeth to provide good coverage for tooth surfaces. Landmarks 400 can also be positioned over teeth 420 relative to features of teeth 420, such as edges between adjacent teeth, proximate the gum line, or proximate the apex of each tooth. FIG. 8A through FIG. 8C illustrate dots 402 as being visible over teeth 420 in the illustrated close-up view. However, dots 402 can be configured to be small as discussed above so as to be not visible from an onlooker at a distance, but that can be perceived in photographic images taken at close range. Additionally, dots 402 can be transparent, e.g., bumps or depressions, so as to not be noticeable at distance, but that can be perceived in electronic imaging.

As discussed herein, orthodontic aligner tray 410 can be positioned over teeth 420, which may cause localized distortions of orthodontic aligner tray 410 that generate forces to move teeth 420. Distortions of orthodontic aligner tray 410 can be determined via tracking movement of dots 402 in a three-dimensional coordinate system, as shown in FIG. 9.

FIG. 9 is a schematic illustration of landmarks 400 disposed in coordinate system 430, which in the illustrated example can comprise a three-dimensional (3D) Cartesian coordinate system. However, other coordinate systems can be used, such as polar coordinate systems. Coordinate system 430 can comprise x-axis 432, y-axis 434 and z-axis 436. X-axis 432, y-axis 434 and z-axis 436 can converge at origin 438. In examples, coordinate system 430 can tied to a specific location on orthodontic aligner tray 410 as can be provided by a refence point. In examples, any location on orthodontic aligner tray 410 that can be repeatedly found with accuracy can be used, such as left-most or right-most edge of the tray. In examples, a specific feature can be used as a fiducial marker from which to reference origin 438 or changes in movements of landmarks 400. In examples, orthodontic aligner tray 410 can include a depression, protrusion, hole or the like as a reference feature. In examples, the reference feature can comprise a small cross positioned on substrate 412. The reference feature or fiducial marker can be located in an out of the way surface or portion of orthodontic aligner tray 410 so as to not be visible when worn by a user, such as on a lingual surface of orthodontic aligner tray 410.

In examples, the starting shape of orthodontic aligner tray 410 and location of the pattern of dots 402 thereon can be known, such as by analyzing orthodontic aligner tray 350 before application to teeth. A patient can wear orthodontic aligner tray 410 for a period of time. In examples, a patient can move a camera in various positions relative to orthodontic aligner tray 410 to capture images of landmarks 400. The patient can obtain images of orthodontic aligner tray 410 at the beginning of application of the aligner tray to the teeth and at the end of the application of the aligner tray to the teeth. Before and after images from the same angle can be compared to each other to determine changes in the shape of the aligner tray have occurred. Computer modeling or simulations can be run to simulate orthodontic aligner tray 410 being applied to the teeth of the patient to move the teeth into the desired position according to a treatment plan at the end of the use of orthodontic aligner tray 410. The simulation can produce expected changes in the shape of orthodontic aligner tray 410 and the associated position of landmarks 400 thereon when the teeth are moved to the desired position. Such simulated or calculated positioning of landmarks 400 can be compared to the changes in position of landmarks 400 obtained from images of the patient wearing orthodontic aligner tray 410. Thus, differences between the simulated or calculated locations of landmarks 400 and the actual locations of landmarks 400 can be used to determine if the teeth have been moved into the desired position. If the teeth have been moved into the desired position, the simulated or calculated locations of landmarks 400 and the actual locations of landmarks 400 will be the same or similar. If the teeth have not moved into the desired position, the simulated or calculated position changes of landmarks 400 changes and the actual position changes of landmarks 400 will be different. As such, a treatment plan can be updated to produce the next stage orthodontic aligner tray based on where orthodontic aligner tray 410 actually moved the teeth. The treatment plan can include a start location and a finish location per stage of aligner tray. This treatment plan can be developed based the material of the aligner tray and can be determined using judgment of the orthodontist or can be determined via algorithm of using computational engine 612 and learning algorithm 615 (FIG. 15).

FIG. 10 is a schematic illustration of orthodontic aligner tray 350 having light diffraction capabilities that can be used to perform tooth position tracking according to the present disclosure. Orthodontic aligner tray 350 can be positioned proximate to light source 352, which can emit light 354 toward orthodontic aligner tray 350. In examples, light source 352 can be light from a smart phone applied by a patient. Simultaneously, a camera of the smartphone can be used to capture images of orthodontic aligner tray 350 while light 354 is shining on the orthodontic aligner tray 350. Light 354 can pass through the material of orthodontic aligner tray 350 and be emitted as spectrum 356 and reflected as spectrum 357. That is, different light spectrum can be reflected than what is refracted and passed through. Spectrum 357 can be captured in photographic digital image of orthodontic aligner tray 350 while light 354 is shining on orthodontic aligner tray 350. In examples, light 354 can comprise white light focused onto particular areas of interest on orthodontic aligner tray 350. Spectrum 356 that is passed through and spectrum 357 that is reflected can have different properties based on the stress and/or strain present within the material of orthodontic aligner tray 350. Some light will be reflected based on internal strains, acting under similar principles as a prism. Thus, before orthodontic aligner tray 350 is worn by a user, spectrum 357 can indicate low stress within the material. For example, the plastic material used to fabricate orthodontic aligner tray 350 can be subjected to confining stress and strain after thermoforming. However, after orthodontic aligner tray 350 is worn by a user, spectrum 357 can indicate high stress within the material at various locations. The adjusted position of the teeth can change the stress induced in the material. These stresses impact the refraction index of the material leading to a unique band of refraction indices throughout the plastic. The reflected light can be visualized and will be dependent on aligner position i.e., the reflected light pattern will change based upon tooth position and the associated induced stresses and strains in orthodontic aligner tray 350.

FIG. 11A is a schematic illustration of orthodontic aligner tray 350 positioned around tooth 360 to show stress applied to orthodontic aligner tray 350 by tooth 360, with orthodontic aligner tray 350 and tooth 360 in a first relative position. In FIG. 11A, tooth 360 can fit between the walls of orthodontic aligner tray 350 in a slightly misaligned position so that corners of tooth 360 push against orthodontic aligner tray 350. As such, most of material of orthodontic aligner tray 350 indicates a light color 362, such as green, indicating low stress, but where corners of tooth 360 push against orthodontic aligner tray 350, the material of orthodontic aligner tray 350 can be dark color 364, such as red, indicating high stress.

FIG. 11B is a schematic illustration of orthodontic aligner tray 350 positioned around tooth 360 to show stress applied to orthodontic aligner tray 350 by tooth 360, with orthodontic aligner tray 350 and tooth 360 in a second relative position. FIG. 11B can represent the state of tooth 360 after orthodontic aligner tray has been applied to tooth 360 for a period of time. In FIG. 11B, tooth 360 can fit between the walls of orthodontic aligner tray 350 in a slightly rotated position so that corners of tooth 360 are fit between the walls of orthodontic aligner tray 350 in a more aligned position where the corners are pushing against orthodontic aligner tray 350 less. However, in order to obtain the rotation of tooth 360, stress can be induced in the material of orthodontic aligner tray 350, as indicated by dark color 364 shifting position to an end of orthodontic aligner tray 350, with areas of low stress shifted to the inner and outer wall of orthodontic aligner tray 350.

FIG. 11A and FIG. 11B illustrate schematic images that can be obtained by a camera or imaging sensor of a smartphone while light from a light source, e.g., the camera flash of the smartphone, is shining on orthodontic aligner tray being worn by a patient. The images show visualization of what refracted and reflected light by the orthodontic aligner tray can look like. The refracted light can be of different wavelengths, e.g., colors, based on the strain present in the material. The color patterns in the images can be analyzed to determine changes in tooth movements due to application of orthodontic aligner tray 350. For example, the starting shape of orthodontic aligner tray 350, the strain present therein and the associated spectrum of color within orthodontic aligner tray 350 can be known, such as by analyzing orthodontic aligner tray 350 before application to teeth. In examples, a patient can move a camera in various positions relative to orthodontic aligner tray 350 to capture images while light 354 is shining. The patient can obtain images of orthodontic aligner tray 350 at the beginning of application of the aligner tray to the teeth and at the end of the application of the aligner tray to the teeth. Before and after images from the same angle can be compared to each other to determine changes in the shape of the aligner tray have occurred. Computer modeling or simulations can be run to simulate orthodontic aligner tray 350 being applied to the teeth of the patient to move the teeth into the desired position according to a treatment plan at the end of the use of orthodontic aligner tray 350. The simulation can produce expected strain within orthodontic aligner tray 350 and the associated spectrum therein when the teeth are moved to the desired position. Such simulated or calculated strain and color spectrum can be compared to the actual color spectrum obtained from images of the patient wearing orthodontic aligner tray 350. Thus, differences between the simulated or calculated spectrum and the actual spectrum can be used to determine if the teeth have been moved into the desired position. If the teeth have been moved into the desired position, the simulated or calculated spectrum and the actual spectrum will be the same or similar. If the teeth have not moved into the desired position, the simulated or calculated spectrum changes and the actual shape changes will be different. As such, a treatment plan can be updated to produce the next stage orthodontic aligner tray based on where orthodontic aligner tray 350 actually moved the teeth.

FIG. 12A is a perspective view of orthodontic aligner tray 500 having contour lines 502 that can be used to perform tooth position tracking. In examples, contour lines 502 can be part of the material of orthodontic aligner tray 500, such as ripples within the molded or overlayed material of orthodontic aligner tray 500. Contour lines 502 can change shape as orthodontic aligner tray 500 changes shape from before being applied to straighten teeth to after having applied force to straighten teeth. In additional examples, the material of orthodontic aligner tray 500 can be smooth and contour lines 502 can be lines added to the material of orthodontic aligner tray 500, such as via etching, burnishing or printing, specifically for the purposes of tracking tooth movements through changes in shape of orthodontic aligner tray 500. Contour lines 502 can provide a distinctive shape that allows for changes in shape to be easily detected in photographic images. Furthermore, contour lines 502 can be viewed with a lens, such as a lens having a complimentary pattern to contour lines 502, that allows changes in contour lines 502 to be more readily detected, as discussed with reference to FIG. 13A and FIG. 13B.

FIG. 12B is an example schematic view of slits 510 formed into orthodontic aligner tray 500. Slits 510 can comprise gaps in the material of orthodontic aligner tray 500 that form edges. As such, the transparency of orthodontic aligner tray 500 can be maintained. In examples, slits 510 can be formed with laser cutting techniques. Slits 510 can follow the path of contour lines 502 or can have another shape or pattern. In examples, small portions of slits 510 can be positioned in select locations on orthodontic aligner tray 500 where it is specifically desirable to track shape changes, rather than extending over the entirety of orthodontic aligner tray 500. As discussed with reference to FIG. 13A and FIG. 13B, the contour lines or slit lines within orthodontic aligner tray 500 can be used to track changes in the shape of orthodontic aligner tray 500, thereby also allowing for tooth movement tracking. In examples, slits 510 can comprise indents within orthodontic aligner tray 500.

FIG.13A is a schematic illustration of orthodontic aligner tray surface 520 having first pattern of lines 522 overlayed with lens 524 having second pattern of lines 526. FIG. 13B is a schematic illustration of orthodontic aligner tray surface 520 of FIG. 13A with orthodontic aligner tray surface 520 translated to show changes in a pattern formed by the overlap of first pattern of lines 522 and second pattern of lines 526. First pattern of lines 522 can comprise lines 528 and gaps 530. Second pattern of lines 526 can comprise lines 532 and gaps 534. Gaps 534 of second pattern of lines 526 can allow for viewing of first pattern of lines 522 behind lens 524. Lines 532 can be disposed over lines 528 to form a pattern of windows 536. In the illustrated examples, windows 536 have diamond shapes. FIG. 13A and FIG. 13B are discussed concurrently.

Orthodontic aligner tray surface 520 can comprise a layer of material of an orthodontic aligner tray. First pattern of lines 522 can be fixedly attached to orthodontic aligner tray surface 520 so that lines 528 and gaps 530 do not move relative to orthodontic aligner tray surface 520. However, lines 528 can change shape, e.g., three-dimensional position, due to changes in the shape of orthodontic aligner tray surface 520.

Lens 524 can comprise a lens for a camera or a filter for a camera. Second pattern of lines 526 can be fixedly attached to lens 524 so that lines 532 and gaps 534 do not move relative to lens 524. However, relative movement between lens 524 and orthodontic aligner tray surface 520 can cause relative movement between second pattern of lines 526 and first pattern of lines 522 that causes the shape or position of windows 536 to change.

First pattern of lines 522 can be positioned over the entirety of an orthodontic aligner tray or over only small portions of an orthodontic aligner tray in selected locations where deformation is likely to occur.

The illustrated example discloses patterns of lines overlapping with each other. However, other patterns can be used. For example, patterns of squares, circles, diamonds, stars and the like can be used. For example, the outlines of circles with the centers open to allow viewing therethrough can be connected to adjacent circles at tangent points. In the illustrated example, both the orthodontic aligner tray and the lens have the same pattern. However, in other examples, the pattern on the orthodontic aligner tray can be different.

In examples, a patient can move a camera having lens 524 in various positions relative to orthodontic aligner tray surface 520 to capture images of second pattern of lines 526 relative to first pattern of lines 522. The patient can obtain images of orthodontic aligner tray surface 520 at the beginning of application of the aligner tray to the teeth and at the end of the application of the aligner tray to the teeth. Before and after images from the same angle can be compared to each other to determine changes in the shape of the aligner tray have occurred. Computer modeling or simulations can be run to simulate orthodontic aligner tray surface 520 being applied to the teeth of the patient to move the teeth into the desired position according to a treatment plan at the end of the use of orthodontic aligner tray surface 520. The simulation can produce expected changes in first pattern of lines 522 on orthodontic aligner tray surface 520 when the teeth are moved to the desired position. Such simulated or calculated shape changes can be compared to the actual shape changes obtained from images of the patient wearing the aligner tray having orthodontic aligner tray surface 520. Thus, differences between the simulated or calculated shape changes and the actual shape changes can be used to determine if the teeth have been moved into the desired position. If the teeth have been moved into the desired position, the simulated or calculated shape changes and the actual shape changes will be the same or similar. If the teeth have not moved into the desired position, the simulated or calculated shape changes and the actual shape changes will be different. As such, a treatment plan can be updated to produce the next stage orthodontic aligner tray based on where orthodontic aligner tray 500 actually moved the teeth.

FIG. 14 is a perspective view of orthodontic aligner tray 550 having sensor 552 that can be used to perform tooth position tracking via tracking changes in the shape of orthodontic aligner tray 550. Sensor 552 can comprise a device configured to determine changes in the shape of orthodontic aligner tray 550 or other parameters or characteristics of orthodontic aligner tray 550. In examples, sensor 552 can comprise a strain gauge that can sense local stretching or contracting of orthodontic aligner tray 550. In examples, sensor 552 can comprise a pressure sensor that can sense local stretching or contracting of orthodontic aligner tray 550. In examples, sensor 552 can be located in an out-of-the-way location on orthodontic aligner tray 550 so as to not be visible when orthodontic aligner tray 550 is in the mouth of a user. Thus, sensor 552 can sense local deformations of orthodontic aligner tray 550 as selected landmark locations where it is desirable to determine tooth position changes. As with other examples, simulations and computer modeling can be run to determine the amount of local strain, stress and/or pressure that should occur in orthodontic aligner tray 550 to induce the desired amount of tooth movement. The simulation and modeling results can be compared to actual sensor output to determine if appropriate tooth movement has occurred according to the treatment plan. In examples, a smartphone can be used to transmit sensor signals to a dental facility that is determining the treatment plan to monitor the effectiveness of orthodontic aligner tray 550. In examples, photographs of orthodontic aligner tray 550 can be obtained by a patient that include sensor 552, wherein the photographs can include output of sensor 552.

FIG. 15 is a schematic illustration of system 600 for determining changes in shape of orthodontic aligner tray 602. System 600 can comprise computing device 604, network 606, communication device 608, image reader 610, computational engine 612, output device 614 and learning algorithm 615. Computing device 604, which in examples can comprise a mobile phone, can comprise light emitter 616, imaging sensor 618, computer 620 and communication device 622. Light emitter 616 can be configured to emit light beam 624. Imaging sensor 618 can be configured to have field of view 626. In additional examples, system 600 can include manufacturing system 628 that can receive manufacturing information from computational engine 612 to fabricate orthodontic aligner tray 602 according to a treatment plan and to include the landmarks, tracking markers and features described herein. System 600 can be used to place landmarks on orthodontic aligner tray 602 during manufacturing, recognize the presence of landmarks on orthodontic aligner tray 602 from digital images, determine a change in position of the landmarks in three-dimensional space relative to the originally manufactured position, and correlate such changes in the position of the landmarks to changes in the position of teeth that have deformed orthodontic aligner tray 602 via wearing for a period of time.

Orthodontic aligner tray 602 can comprise any of the orthodontic aligner trays described herein. Orthodontic aligner tray 602 can include any of the landmarks, landmark locations, material properties, markers or sensors discussed herein. Orthodontic aligner tray 602 can be provided to a patient so that the patient can wear orthodontic aligner tray 602 according to a treatment plan, such as treatment plan 630, for a period of time. Treatment plan 630 can be developed as described herein.

Computing device 604 can comprise any computing device capable of obtaining photographic, e.g., digital images, of orthodontic aligner tray 602. As such, computing device 604 can include imaging sensor 618. In examples, imaging sensor 618 can comprise a charge-coupled device (CCD), a solid state device such as a complementary metal oxide semiconductor (CMOS), a focal plane array (FPA) imaging sensor, another imaging sensor or camera or another device known in the art. In examples, imaging sensor 618 can include multiple imaging sensors to facilitate obtaining digital images having three-dimensional aspects, e.g., wherein software of computing device 604 can merge multiple images from different angels to include three-dimensional depth data.

Computing device 604 can additionally include light emitter 616, which can be configured to emit light onto orthodontic aligner tray 602. In examples, light emitter 616 can comprise one or more light emitting diodes (LEDs). In examples, other types of light emitters can be used such as incandescent light bulbs and fluorescent light bulbs. In examples, light emitter 616 can be configured to emit white light. However, light emitter 616 can be configured to emit light of other wavelengths, such as ultraviolet light, infrared light, blue light and the like.

In examples, computing device 604 can include hardware and software suitable for processing images obtained by imaging sensor 618. In examples, computing device 604 can comprise computer 620, which can comprise a central processing unit and memory. In examples, computer 620 can include computer readable storage medium including instructions for forming and interpreting images obtained by imaging sensor 618. In examples, imaging sensor 618 can comprise machine-readable media including: non-volatile memory, such as semiconductor memory devices (e.g., Electrically Programmable Read-Only Memory (EPROM), Electrically Erasable Programmable Read-Only Memory (EPSOM)) and flash memory devices; magnetic disks, such as internal hard disks and removable disks; magneto-optical disks; and CD-ROM and DVD-ROM disks.

Communication device 622 can comprise an electronic communication device suitable for transmitting digital files of digital images obtained by imaging sensor 618 electronically. In examples, communication device 622 can comprise can be capable of transmitting over a number of different transfer protocols (e.g., frame relay, internet protocol (IP), transmission control protocol (TCP), user datagram protocol (UDP), hypertext transfer protocol (HTTP), etc.). Example communication networks can include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as WiFi^{®}, IEEE 802.16 family of standards known as WiMax^{®}), IEEE 802.15.4 family of standards, peer-to-peer (P2P) networks, Bluetooth, Zigbee, infrared (IR), near field communication (NFC), 3GPP or other technologies Bluetooth, among others. In an example, communication device 622 can include one or more physical jacks (e.g., Ethernet, coaxial, or phone jacks) or one or more antennas to connect to network 606. In an example, communication device 622 can include a plurality of antennas to wirelessly communicate using at least one of single-input multiple-output (SIMO), multiple-input multiple-output (MIMO), or multiple-input single-output (MISO) techniques.

Communication device 622 can transmit data files including images of orthodontic aligner tray 602 to network 606. Network 606 can comprise a server machine, a client machine, or both in server-client network environments. In an example, network 606 can act as a peer machine in peer-to-peer (P2P) (or other distributed) network environment. Network 606 can be in whole or part a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Network 606 can additionally be in communication with communication device 608. Communication device 608 can be configured as described herein, such as with respect to communication device 622. In examples, communication device 622 can be part of a computer system including image reader 610, computational engine 612 and output device 614, which can be located at a dental office, a manufacturing facility and other locations. Image files received by communication device 608 can be communicated to image reader 610.

Image reader 610 can be configured to identify the locations of landmarks, markers, material properties and the like within the image files related to orthodontic aligner tray 602. Image reader 610 can comprise a computing system that can use image recognition software and programs to locate the landmarks, markers and material properties in the image files. Image reader 610 can transmit the image files including the identified landmarks, markers and material properties to computational engine 612.

Computational engine 612 can comprise a computer capable of executing mathematical software, such as CAD programs, finite element analysis (FEA) software, mathematical computation engines and the like. Computational engine 612 can be configured to operate the Ortho Analyzer Next Generation software from 3shape or CEREC Ortho SW 2.0 software from Dentsply Sirona. Computational engine 612 can be configured to determine the location of the landmarks, markers and material properties in three-dimensional space, such as coordinate system 430 of FIG. 9. Computational engine 612 can be configured to triangulate the position of specific landmarks using multiple images within a three-dimensional coordinate system. Computational engine 612 can determine difference in positions of landmarks on a used orthodontic aligner tray compared to an unused orthodontic aligner tray, translate those differences into changes in teeth positions based on performance of the orthodontic aligner tray, compare the changes in position of the teeth to planned changes in position of the teeth for the orthodontic aligner tray based on a previously determined treatment plan, determine if a next stage orthodontic aligner tray is compatible with the current position of the teeth, and, if necessary, update shape information for the next stage orthodontic aligner tray to receive the teeth in their current position and to change the current position of the teeth into another position along the treatment plan. Computational engine 612 can be used in conjunction with learning algorithm 615 to analyze landmarks and determine the shape of subsequent aligner trays. In examples, learning algorithm 615 can comprise a machine learning model. Learning algorithm 615 can be trained on image data of aligner trays including landmarks before and after being worn to make predictions or decisions without explicit programming, learning from patterns in images of landmarks and improving performance of itself over time as more image data is added to its database. Learning algorithm 615 can include supervised models, unsupervised models an reinforcement learning models.

Output device 614 can be configured to receive output of mathematical computations from computational engine 612 and provide output to a user. Output device 614 can comprise display screens, video monitors, speakers, audio and visual alarms and data transmission devices. Output device 614 can include or comprise a device for making waves, such as a sound wave or a vibration wave, such as an auditory device, a speaker or amplifier for producing an auditory signal or sound. Output device 614 can comprise devices for providing visual and audio feedback. Output device 614 can comprise a device for producing light waves, such as incandescent light bulbs, light-emitting-diodes and the like. Output device 614 can comprise various input/output (I/O) devices, such as buttons, knobs, dials, a keyboard, a mouse, touchscreens, touch pads, microphones, video cameras, imaging devices, scanners and sensors (e.g., light sensors, position sensors, speed sensors, proximity sensors, imaging sensors). Output device 614 can allow a user, e.g., a dental professional, view in formation related to changes in shape of orthodontic aligner tray 602. For example, output device 614 can display images similar to those of FIG. 2 through FIG. 6B, FIG. 9, FIG. 11A, FIG. 11B, FIG. 13A and FIG. 13B. Thus, output device 614 can thus be configured to provide visualization of orthodontic aligner trays before and after being worn by a patient for a period of time and deviations of the actual change from the expected change, such as by using three-dimensional point clouds, three-dimensional vectors, heat maps and the like that can allow a dental professional to determine how close the orthodontic aligner tray was to achieving the planned change in position of one or more teeth, thereby allowing the user and computational engine 612 to determine if the treatment plan should be updated and the shape of a new orthodontic aligner tray to continue the treatment plan.

Manufacturing system 628 can comprise any manufacturing system suitable for fabricating orthodontic aligner tray 602. In examples, manufacturing system 628 can be thermo-formed over a physical overlay model using, for example, an Essix vacuum forming machine and Essix Ace plastic thermoforming sheets. Both the sheets and the thermoforming machine are commercially available from Dentsply Sirona, Inc. In examples, the thermoforming process can comprise conventional and known processes in the dental art. In other examples, the aligner trays can be fabricated using other manufacturing methods, such as additive manufacturing processes and three-dimensional printing processes. Manufacturing system 628 can be configured to add paint layers or ink layers to orthodontic aligner tray 602 to produce landmarks 400. In examples, manufacturing system 628 can be configured to add dimples, bumps or slits to orthodontic aligner tray 602. In examples, manufacturing system 628 can add sensor 552 to orthodontic aligner tray 602. Manufacturing system 628 can include post-processing, such as edge cutting, such as by using laser cutting systems, and final wash, such as by using hot water, Isopropyl Alcohol (IPA), etc.

The present disclosure provides systems and methods for tracking changes in tooth position resulting from the application of orthodontic aligner trays by tracking the position of landmarks, markers and material properties of the orthodontic aligner trays from the beginning of the application of the orthodontic aligner tray to the end of the orthodontic aligner tray. The changes in shape of the orthodontic aligner trays from the beginning to end can correlate to changes in tooth position, which can be compared to intended or designed changes in tooth position. Thus, the shape of the succeeding orthodontic aligner tray can be better planned and manufactured to continue the treatment plan. Overall, the systems and methods of the present disclosure can improve treatment plans from orthodontic aligner trays by reducing the occurrence of having to remanufacture outdated orthodontic aligner trays. Features and benefits of the present disclosure include:

Landmarks on Aligner Trays: The present disclosure relates to the incorporation of a plurality of landmarks, such as small dots, on the aligner trays. These landmarks can be configured to change configuration commensurate with changes in the shape of the tray, allowing for tracking of tooth movement.

Digital Detection: The landmarks can be digitally detectable, enabling tracking through digital photographic images. This allows for convenient monitoring of tooth movement without the need for frequent office visits.

Feedback Mechanism: The system provides feedback on the performance of each aligner tray, allowing for mid-course corrections to the treatment plan if necessary. This helps in reducing waste and improving the efficiency of the treatment process.

Material Properties: The aligner trays can include material properties that change under stress, such as light diffraction capabilities, which can be used to track changes in teeth positions.

Contour Lines and Patterns: The aligner trays can feature contour lines, slits, or Moiré patterns that assist in visualizing changes in the tray's shape, further aiding in the tracking process.

Sensors: The present disclosure can include sensors embedded in the aligner trays to detect forces applied to the teeth, providing additional data for tracking tooth movement.

Convenience for Patients: The system allows patients to capture images of the aligner trays at home, reducing the need for in-office visits and making the monitoring process more convenient.

Reduced Waste: The present disclosure can eliminate the production of unused orthodontic aligner trays that are pre-produced according to a treatment plan, but that cannot be used because treatment of the teeth has not progressed as expected.

### Examples

Example 1 is an orthodontic aligner tray comprising: a shell having a plurality of tooth receiving cavities shaped to apply a repositioning force to teeth of a patient; and a plurality of landmarks present on the shell, wherein the plurality of landmarks are configured to be detected to determine a shape of the shell.

In Example 2, the subject matter of Example 1 optionally includes wherein the plurality of landmarks are configured to change configuration commensurate with changes in shape of the shell.

In Example 3, the subject matter of any one or more of Examples 1-2 optionally include wherein changes in the shape of the shell are induced by movement of the teeth of the patient.

In Example 4, the subject matter of any one or more of Examples 1-3 optionally include wherein landmarks of the plurality of landmarks are located at landmark positions on the shell to track rotational and translational movement of individual teeth.

In Example 5, the subject matter of any one or more of Examples 1-4 optionally include wherein the plurality of landmarks is digitally detectable to track the changes in the shape of the shell.

In Example 6, the subject matter of any one or more of Examples 1-5 optionally include wherein the plurality of landmarks is optically detectable.

In Example 7, the subject matter of any one or more of Examples 5-6 optionally include wherein the plurality of landmarks is detectable in digital photographic images.

In Example 8, the subject matter of any one or more of Examples 5-7 optionally include wherein the plurality of landmarks is detectable in a digital scan of the orthodontic aligner tray.

In Example 9, the subject matter of any one or more of Examples 5-8 optionally include wherein the plurality of landmarks comprises an array of marks distributed over a surface of the shell.

In Example 10, the subject matter of Example 9 optionally includes wherein each landmark of the plurality of landmarks has a size in a range of approximately five microns to approximately two-hundred microns.

In Example 11, the subject matter of Example 10 optionally includes wherein the plurality of landmarks comprises a plurality of dots.

In Example 12, the subject matter of Example 11 optionally includes wherein dots of the plurality of dots have varying size.

In Example 13, the subject matter of any one or more of Examples 11-12 optionally include wherein dots of the plurality of dots are arranged in a grid pattern having equidistant spacing.

In Example 14, the subject matter of any one or more of Examples 11-13 optionally include wherein dots of the plurality of dots are arranged in an asymmetric pattern.

In Example 15, the subject matter of any one or more of Examples 5-14 optionally include wherein at least some of the plurality of landmarks comprise markings added to one or more surfaces of the shell.

In Example 16, the subject matter of Example 15 optionally includes wherein the markings comprise biocompatible ink applied to exterior surfaces of the shell.

In Example 17, the subject matter of any one or more of Examples 15-16 optionally include wherein the markings comprise depressions formed into exterior surfaces of the shell.

In Example 18, the subject matter of any one or more of Examples 5-17 optionally include wherein material of the shell includes a plurality of contour lines detectable in a photographic image.

In Example 19, the subject matter of Example 18 optionally includes wherein the plurality of contour lines comprise ripples within the material of the shell.

In Example 20, the subject matter of any one or more of Examples 18-19 optionally include wherein the plurality of contour lines comprise slits within material of the shell.

In Example 21, the subject matter of any one or more of Examples 18-20 optionally include wherein the plurality of contour lines comprise contours or indents.

In Example 22, the subject matter of any one or more of Examples 1-21 optionally include wherein the plurality of landmarks is detectable using illumination.

In Example 23, the subject matter of Example 22 optionally includes wherein the plurality of landmarks comprises invisible ink that can be read when illuminated with light.

In Example 24, the subject matter of any one or more of Examples 22-23 optionally include wherein the plurality of landmarks comprise microstructure of material of the shell at specific landmark locations.

In Example 25, the subject matter of Example 24 optionally includes wherein: the shell comprises a polymer material that undergoes birefringence; and the polymer material exhibits optical properties that change under applied stress.

In Example 26, the subject matter of Example 25 optionally includes wherein the plurality of landmarks is configured to be detected using white light.

In Example 27, the subject matter of any one or more of Examples 25-26 optionally include wherein the plurality of landmarks is configured to be detected using a specific wavelength of light.

In Example 28, the subject matter of any one or more of Examples 1-27 optionally include at least one sensor disposed on the shell configured to detect forces resulting from change in shape of the orthodontic aligner tray.

Example 29 is a method of tracking movement of teeth from application of a first orthodontic aligner tray, the method comprising: designing the first orthodontic aligner tray to have a plurality of landmarks disposed at a first landmark position within a three-dimensional coordinate system for the first orthodontic aligner tray; receiving electronic files of the first orthodontic aligner tray after being applied to teeth so that the first orthodontic aligner tray is deformed to move at least some of the plurality of landmarks into a second landmark position; determining changes in locations of the plurality of landmarks in the three-dimensional coordinate system from the first landmark position to the second landmark position from the electronic files; and outputting an indication of the changes.

In Example 30, the subject matter of Example 29 optionally includes correlating a change from the first landmark position to the second landmark position to a change in at least one tooth from a first tooth position to a second tooth position.

In Example 31, the subject matter of Example 30 optionally includes generating: a treatment plan for changing position of at least a first tooth with the first orthodontic aligner tray from the first tooth position to a third tooth position; and designing a second orthodontic aligner tray to move the first tooth from the second tooth position to the third tooth position.

In Example 32, the subject matter of Example 31 optionally includes wherein the treatment plan further comprises a planned shape for the second orthodontic aligner tray to move the teeth to the third tooth position.

In Example 33, the subject matter of Example 32 optionally includes wherein outputting the indication of the changes comprises outputting electronic images on a display screen showing: images of the first orthodontic aligner tray that is deformed so the plurality of landmarks are in the second landmark position; and a model of the first orthodontic aligner tray with the plurality of landmarks in the planned shape.

In Example 34, the subject matter of any one or more of Examples 29-33 optionally include wherein outputting the indication of the changes comprises outputting electronic images on a display screen showing: a vector indicating a difference in actual position versus expected position for a plurality of landmark locations of the first orthodontic aligner tray.

In Example 35, the subject matter of any one or more of Examples 29-34 optionally include wherein determining changes in locations of the plurality of landmarks in the three-dimensional coordinate system comprises: imaging the first orthodontic aligner tray to obtain a first image set comprising the electronic files; and determining changes in position of the plurality of landmarks in the first image set with a computer system based on comparison to an initial image set of the first orthodontic aligner tray with the plurality of landmarks disposed at the first landmark position.

In Example 36, the subject matter of any one or more of Examples 29-35 optionally include wherein receiving electronic files of the first orthodontic aligner tray comprises receiving a plurality of photographic images of the teeth wearing the first orthodontic aligner tray.

In Example 37, the subject matter of Example 36 optionally includes receiving the plurality of photographic images of the teeth wearing the first orthodontic aligner tray from a patient having the teeth.

In Example 38, the subject matter of any one or more of Examples 29-37 optionally include wherein receiving electronic files of the first orthodontic aligner tray comprises: receiving the first orthodontic aligner tray from a patient having the teeth; and imaging the first orthodontic aligner tray.

In Example 39, the subject matter of any one or more of Examples 29-38 optionally include wherein receiving electronic files of the first orthodontic aligner tray comprises receiving a three-dimensional model of the teeth wearing the first orthodontic aligner tray generated by a three-dimensional scanner.

In Example 40, the subject matter of any one or more of Examples 29-39 optionally include wherein determining changes in locations of the plurality of landmarks in the three-dimensional coordinate system comprises: determining changes in position of a matrix of markers on the first orthodontic aligner tray.

In Example 41, the subject matter of Example 40 optionally includes shining light on the matrix of markers to allow the matrix of markers to be visible.

In Example 42, the subject matter of any one or more of Examples 29-41 optionally include wherein determining changes in locations of the plurality of landmarks in the three-dimensional coordinate system comprises: illuminating material within the first orthodontic aligner tray at landmark locations; and reflecting light via birefringence at the landmark locations to reveal stress concentration.

In Example 43, the subject matter of any one or more of Examples 29-42 optionally include wherein determining changes in locations of the plurality of landmarks in the three-dimensional coordinate system comprises: viewing a plurality of contour lines on the first orthodontic aligner tray; and moving a lens having a complimentary plurality of contour lines across the plurality of contour lines.

In Example 44, the subject matter of Example 43 optionally includes wherein the plurality of contour lines comprise curved slits within the first orthodontic aligner tray.

In Example 45, the subject matter of any one or more of Examples 29-44 optionally include wherein determining changes in locations of the plurality of landmarks in the three-dimensional coordinate system comprises: receiving feedback from a sensor located at a landmark location on the first orthodontic aligner tray.

In Example 46, the subject matter of Example 45 optionally includes wherein the sensor comprises one or more of a strain gauge and a pressure sensor.

Example 47 is a method for tracking progress of orthodontic treatment using an aligner tray, the method comprising: providing an aligner tray having a plurality of markers disposed thereon; positioning the aligner tray on teeth of a patient; capturing image data of the aligner tray; analyzing the image data to determine positions of the plurality of markers; comparing the determined positions of the plurality of markers with expected positions based on a treatment plan; determining whether the teeth are moving according to the treatment plan based on the comparison; and revising the treatment plan when the comparison indicates that the teeth are not moving according to the treatment plan.

In Example 48, the subject matter of Example 47 optionally includes wherein revising the treatment plan comprises generating a new set of aligner trays before completion of a current set of aligner trays.

In Example 49, the subject matter of any one or more of Examples 47-48 optionally include wherein the capturing of the imaging data comprises the patient obtaining images of the aligner tray.

In Example 50, the subject matter of Example 49 optionally includes wherein the capturing of the imaging data comprises the patient obtaining images of the aligner tray using a handheld camera device.

In Example 51, the subject matter of any one or more of Examples 47-50 optionally include wherein the capturing of the imaging data comprises a dental professional obtaining images of the aligner tray.

In Example 52, the subject matter of Example 51 optionally includes wherein the capturing of the imaging data comprises the dental professional obtaining images of the aligner tray using a scanner.

In Example 53, the subject matter of any one or more of Examples 47-52 optionally include wherein the capturing of the image data of the aligner tray is obtained while the aligner tray is positioned on the teeth.

In Example 54, the subject matter of any one or more of Examples 47-53 optionally include wherein the capturing of the image data of the aligner tray is obtained while the aligner tray is removed from the teeth after being applied to the teeth for a period of time.

In Example 55, the subject matter of any one or more of Examples 47-54 optionally include wherein revising the treatment plan comprises generating a new set of aligner trays before completion of a current set of aligner trays.

In Example 56, the subject matter of any one or more of Examples 1-55 optionally include transmitting the captured image data to a remote server for analysis.

Each of these non-limiting examples can stand on its own, or can be combined in various permutations or combinations with one or more of the other examples.

### Various Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventor also contemplates examples in which only those elements shown or described are provided. Moreover, the present inventor also contemplates examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. An orthodontic aligner tray comprising:
a shell having a plurality of tooth receiving cavities shaped to apply a repositioning force to teeth of a patient; and
a plurality of landmarks present on the shell, wherein the plurality of landmarks are configured to be detected to determine a shape of the shell.

2. The orthodontic aligner tray of claim **1,** wherein:
the plurality of landmarks are configured to change configuration commensurate with changes in shape of the shell; and
changes in the shape of the shell are induced by movement of the teeth of the patient.

3. The orthodontic aligner tray of claim **1,** wherein landmarks of the plurality of landmarks are located at landmark positions on the shell to track rotational and translational movement of individual teeth.

4. The orthodontic aligner tray of claim **1,** wherein the plurality of landmarks is digitally or optically detectable or detectable in digital photographic images or scans to track changes in the shape of the shell.

5. The orthodontic aligner tray of claim 4, wherein the plurality of landmarks comprises a plurality of dots arranged in a grid pattern having equidistant or asymmetric spacing.

6. The orthodontic aligner tray of claim 4, wherein at least some of the plurality of landmarks comprise markings added to one or more surfaces of the shell;
optionally wherein the markings comprise depressions formed into exterior surfaces of the shell.

7. The orthodontic aligner tray of claim 4, wherein material of the shell includes a plurality of contour lines or slits detectable in a photographic image.

8. The orthodontic aligner tray of claim **1,** wherein the plurality of landmarks is detectable using illumination, and:
the plurality of landmarks comprises invisible ink that can be read when illuminated with light; or
the shell comprises a polymer material that undergoes birefringence, wherein the polymer material exhibits optical properties that change under applied stress.

9. The orthodontic aligner tray of claim **1,** further comprising at least one sensor disposed on the shell configured to detect forces resulting from change in shape of the orthodontic aligner tray.

10. A method of tracking movement of teeth from application of a first orthodontic aligner tray, the method comprising:
designing the first orthodontic aligner tray to have a plurality of landmarks disposed at a first landmark position within a three-dimensional coordinate system for the first orthodontic aligner tray;
receiving electronic files of the first orthodontic aligner tray after being applied to teeth so that the first orthodontic aligner tray is deformed to move at least some of the plurality of landmarks into a second landmark position;
determining changes in locations of the plurality of landmarks in the three-dimensional coordinate system from the first landmark position to the second landmark position from the electronic files; and
outputting an indication of the changes.

11. The method of claim 10, further comprising correlating a change from the first landmark position to the second landmark position to a change in at least one tooth from a first tooth position to a second tooth position.

12. The method of claim **11,** further comprising generating:
a treatment plan for changing position of at least a first tooth with the first orthodontic aligner tray from the first tooth position to a third tooth position; and
designing a second orthodontic aligner tray to move the first tooth from the second tooth position to the third tooth position;
wherein the treatment plan further comprises a planned shape for the second orthodontic aligner tray to move the teeth to the third tooth position;
optionally wherein outputting the indication of the changes comprises outputting electronic images on a display screen showing:
images of the first orthodontic aligner tray that is deformed so the plurality of landmarks are in the second landmark position; and
a model of the first orthodontic aligner tray with the plurality of landmarks in the planned shape.

13. The method of claim 10, wherein receiving electronic files of the first orthodontic aligner tray comprises receiving a plurality of photographic images of the teeth wearing the first orthodontic aligner tray from a patient having the teeth.

14. The method of claim 10, wherein determining changes in locations of the plurality of landmarks in the three-dimensional coordinate system comprises:
illuminating material within the first orthodontic aligner tray at landmark locations, and reflecting light via birefringence at the landmark locations to reveal stress concentration;
viewing a plurality of contour lines on the first orthodontic aligner tray, and moving a lens having a complimentary plurality of contour lines across the plurality of contour lines; or
receiving feedback from a sensor located at a landmark location on the first orthodontic aligner tray.

15. A method for tracking progress of orthodontic treatment using an aligner tray, the method comprising:
providing an aligner tray having a plurality of markers disposed thereon;
positioning the aligner tray on teeth of a patient;
capturing image data of the aligner tray;
analyzing the image data to determine positions of the plurality of markers;
comparing the determined positions of the plurality of markers with expected positions based on a treatment plan;
determining whether the teeth are moving according to the treatment plan based on the comparison; and
revising the treatment plan when the comparison indicates that the teeth are not moving according to the treatment plan.
